# EUROPÄISCHE PATENTSCHRIFT

(11) **EP 1 490 342 B1**
(45) Veröffentlichungstag und Bekanntmachung des Hinweises auf die Patenterteilung: **15.12.2010**
(21) Anmeldenummer: 03742451.2
(22) Anmeldetag: 06.02.2003
(51) Int. Cl.: C07D 231/14, A01N 43/56

(54) **DISUBSTITUIERTE PYRAZOLYLCARBOXANILIDE**
DISUBSTITUTED PYRAZOLYL CARBOXANILIDES
CARBOXANILIDES DE PYRAZOLYLE DISUBSTITUES

(30) Priorität: 19.02.2002 DE 10206794; 08.04.2002 DE 10215292
(43) Veröffentlichungstag der Anmeldung: 29.12.2004
(73) Patentinhaber: Bayer CropScience AG, 40789 Monheim (DE)
(72) Erfinder: DUNKEL, Ralf, D-40789 Monheim (DE); RIECK, Heiko, F-69009 Lyon (FR); ELBE, Hans-Ludwig, D-42326 Wuppertal (DE); WACHENDORFF-NEUMANN, Ulrike, D-56566 Neuwied (DE); KUCK, Karl-Heinz, D-40764 Langenfeld (DE)
(86) Internationale Anmeldenummer: PCT/EP2003/001178
(87) Internationale Veröffentlichungsnummer: WO 2003/070705

(56) Entgegenhaltungen:
- EP-A- 0 589 301
- WO-A-00/14071
- WO-A-01/42223
- WO-A-97/08148
- WO-A-99/09013

## Beschreibung

Die vorliegende Erfindung betrifft neue Pyrazolylcarboxanilide, mehrere Verfahren zu deren Herstellung und deren Verwendung zur Bekämpfung von schädlichen Mikroorganismen im Pflanzenschutz und Materialschutz.

Es ist bereits bekannt geworden, dass zahlreiche Carboxanilide fungizide Eigenschaften besitzen (vgl. z.B. EP 0 545 099 und JP 9132567). Die Wirksamkeit der dort beschriebenen Stoffe ist gut, lässt aber bei niedrigen Aufwandmengen in manchen Fallen zu wünschen übrig.

Es wurden nun neue Pyrazolylcarboxanilide der Formel (I) 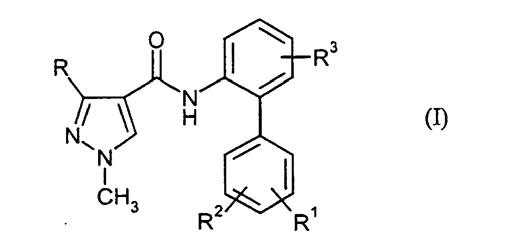 gefunden, in welcher
- R: für Difluormethyl oder Trifluormethyl steht,
- R¹ und R²: unabhängig voneinander für Halogen, Cyano, Nitro, C₁-C₆-Alkyl, C₂-C₆- Alkenyl, C₁-C₄-Alkoxy, C₁-C₄-Alkylthio, C₁-C₄-Alkylsulfonyl, C₃-C₆-Cyclo- alkyl, oder für C₁-C₄-Halogenalkyl, C₁-C₄-Halogenalkoxy, C₁-C₄-Halogen- alkylthio oder C₁-C₄-Halogenalkylsulfonyl mit jeweils 1 bis 5 Halogenatomen stehen,
- R³: für Fluor steht.

Weiterhin wurde gefunden, dass man Pyrazolylcarboxanilide der Formel (I) erhält, indem man
a) Pyrazolylcarbonsäurehalogenide der Formel (II) 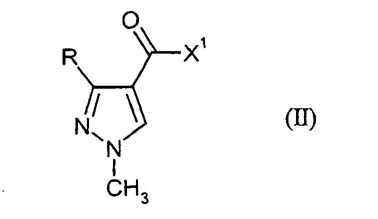 in welcher
   - R: die oben angegebenen Bedeutungen hat,
   - X¹: für Halogen steht,
   mit Anilinderivaten der Formel (III) 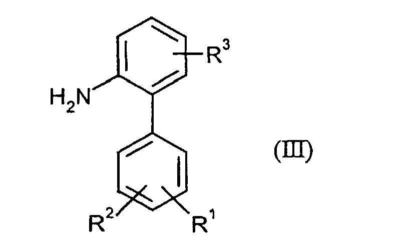 in welcher
   R¹, R² und R³ die oben angegebenen Bedeutungen haben,
   gegebenenfalls in Gegenwart eines Säurebindemittels und gegebenenfalls in Gegenwart eines Verdünnungsmittels umsetzt, oder
b) Halogenpyrazolcarboxanilide der Formel (IV) 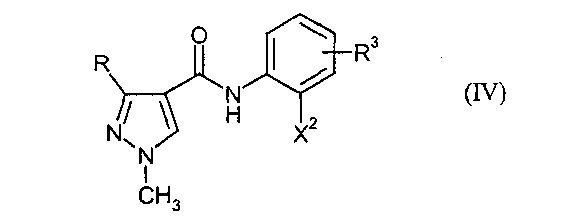 in welcher
   - R und R³: die oben angegebenen Bedeutungen haben,
   - X²: für Brom oder Iod steht,
   mit Boronsäurederivaten der Formel (V) 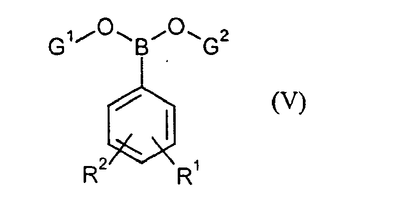 in welcher
   - R¹ und R²: die oben angegebenen Bedeutungen haben,
   - G¹ und G²: jeweils für Wasserstoff oder zusammen für Tetramethylethylen stehen,
   in Gegenwart eines Katalysators, gegebenenfalls in Gegenwart eines Säurebindemittels und gegebenenfalls in Gegenwart eines Verdünnungsmittels umsetzt, oder
c) Halogenpyrazolcarboxanilide der Formel (IV) 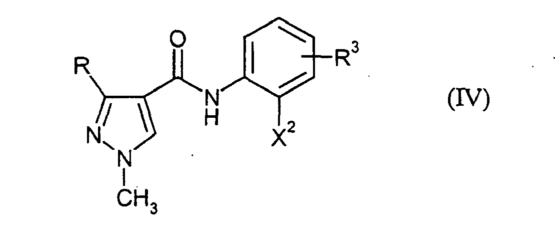 in welcher
   - R und R³: die oben angegebenen Bedeutungen haben,
   - X²: für Brom oder Iod steht,
   in einer ersten Stufe mit einem Diboran-Derivat der Formel (VI) 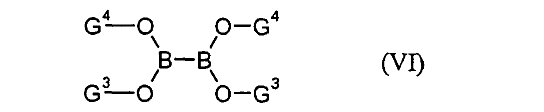 in welcher
   G³ und G⁴ jeweils für Alkyl oder gemeinsam für Alkandiyl stehen, in Gegenwart eines Katalysators, gegebenenfalls in Gegenwart eines Säurebindemittels und gegebenenfalls in Gegenwart eines Verdünnungsmittels umsetzt und ohne Aufarbeitung in einer zweiten Stufe mit Halogenbenzolderivaten der Formel (VII) 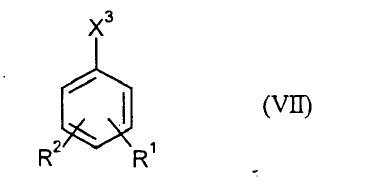 in welcher
   - R¹ und R²: die oben angegebenen Bedeutungen haben und
   - X³: für Brom, Iod oder Trifluormethylsulfonyloxy steht,
   in Gegenwart eines Katalysators, gegebenenfalls in Gegenwart eines Säurebindemittels und gegebenenfalls in Gegenwart eines Verdünnungsmittels umsetzt.

Schließlich wurde gefunden, dass die neuen Pyrazolylcarboxanilide der Formel (I) sehr gute mikrobizide Eigenschaften besitzen und zur Bekämpfung unerwünschter Mikroorganismen sowohl im Pflanzenschutz als auch im Materialschutz verwendbar sind.

Überraschenderweise zeigen die erfindungsgemäßen Pyrazolylcarboxanilide der Formel (I) eine wesentlich bessere fungizide Wirksamkeit als die konstitutionell ähnlichsten, vorbekannten Wirkstoffe gleicher Wirkungsrichtung.

Die erfindungsgemäßen Pyrazolylcarboxanilide sind durch die Formel (I) allgemein definiert.

Bevorzugt sind Pyrazolylcarboxanilide der Formel (I), in welcher
- R: für Difluormethyl oder Trifluormethyl steht,
- R¹ und R²: unabhängig voneinander für Fluor, Chlor, Brom, Cyano, Nitro, Methyl, Ethyl, n- oder i-Propyl, n-, i-, s- oder t-Butyl, Methoxy, Ethoxy, Methylthio, Ethylthio, n- oder i-Propylthio, Cyclopropyl, Trifluormethyl, Trichlormethyl, Trifluorethyl, Difluormethoxy, Trifluormethoxy, Difluorchlormethoxy, Tri- fluorethoxy, Difluormethylthio, Difluorchlormethylthio oder Trifluormethyl- thio stehen,
- R³: für Fluor steht.

Besonders bevorzugt sind Pyrazolylcarboxanilide der Formel (I), in welcher
- R: für Difluormethyl oder Trifluormethyl steht,
- R¹ und R²: unabhängig voneinander für Fluor, Chlor, Brom, Methyl, Trifluormethyl, Difluormethoxy oder Trifluormethoxy stehen,
- R³: für Fluor steht.

Ganz besonders bevorzugt sind Pyrazolylcarboxanilide der Formel (I), in welcher R¹ für Fluor und R² für Chlor steht.

Ganz besonders bevorzugt sind Pyrazolylcarboxanilide der Formel (I), in welcher R¹ für Fluor und R² für Fluor steht.

Ganz besonders bevorzugt sind Pyrazolylcarboxanilide der Formel (I), in welcher R¹ für Methyl oder Trifluormethyl steht.

Gegenstand der Erfindung sind vorzugsweise Verbindungen der Formel (Ia), 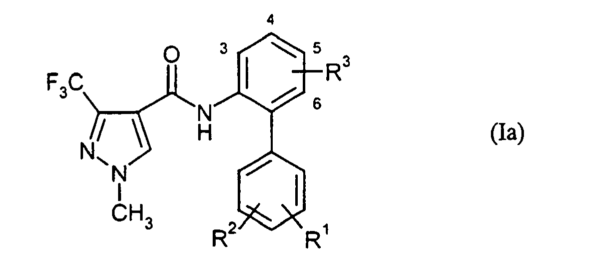 in welcher
- R¹ und R²: unabhängig voneinander für Fluor, Chlor, Brom, Cyano, Nitro, Methyl, Ethyl, n- oder i-Propyl, n-, i-, s- oder t-Butyl, Trifluormethyl, Trichlormethyl, Trifluorethyl, Cyclopropyl, Methoxy, Ethoxy, Difluormethoxy, Trifluor- methoxy, Difluorchlormethoxy, Trifluorethoxy, Methylthio, Ethylthio, n- oder i-Propylthio, Difluormethylthio, Difluorchlormethylthio, Trifluormethylthio stehen und
- R³: für Fluor steht.

Vorzugsweise Gegenstand der vorliegenden Erfindung sind ebenso Verbindungen der Formel (Ib), 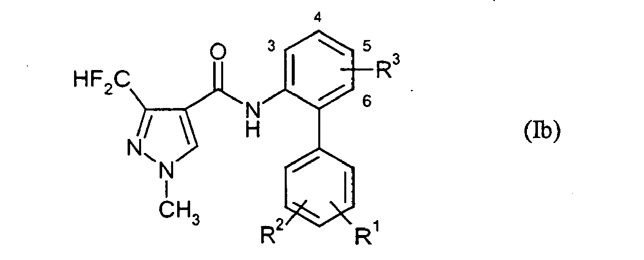 in welcher
- R¹ und R²: unabhängig voneinander für Fluor, Chlor, Brom, Cyano, Nitro, Methyl, Ethyl, n- oder i-Propyl, n-, i-, s- oder t-Butyl, Trifluormethyl, Trichlormethyl, Trifluorethyl, Cyclopropyl, Methoxy, Ethoxy, Difluormethoxy, Trifluor- methoxy, Difluorchlormethoxy, Trifluorethoxy, Methylthio, Ethylthio, n- oder i-Propylthio, Difluormethylthio, Difluorchlormethylthio, Trifluormethylthio stehen und
- R³ für: Fluor steht.

Die vorliegende Anmeldung betrifft insbesondere Verbindungen der Formel (Ia), in welcher
- R¹ und R²: unabhängig voneinander für Fluor, Chlor, Brom, Methyl, Trifluormethyl, Difluormethoxy oder Trifluormethoxy stehen und
- R³: für Fluor steht.

Die vorliegende Anmeldung betrifft ebenso insbesondere Verbindungen der Formel (Ib), in welcher
- R¹ und R²: gleich oder verschieden sind und unabhängig voneinander für Fluor, Chlor, Brom, Methyl, Trifluormethyl, Difluormethoxy oder Trifluormethoxy stehen und
- R³: für Fluor steht.

Die vorliegende Anmeldung betrifft ganz besonders bevorzugt Verbindungen der Formel (Ia), in welcher
- R¹ und R²: unabhängig voneinander für Fluor, Chlor, Brom, Methyl, Trifluormethyl, Difluormethoxy oder Trifluormethoxy stehen und
- R³: für 3-Fluor oder 5-Fluor steht.

Die vorliegende Anmeldung betrifft ebenso ganz besonders bevorzugt Verbindungen der Formel (Ib), in welcher
- R¹ und R²: unabhängig voneinander für Fluor, Chlor, Brom, Methyl, Trifluormethyl, Difluormethoxy oder Trifluormethoxy stehen und
- R³: für 3-Fluor oder 5-Fluor steht.

Außerdem bevorzugt sind Verbindungen der Formel (Ic), 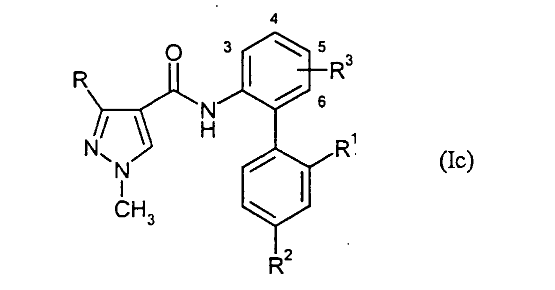 in welcher
- R: für Difluormethyl oder Trifluormethyl steht,
- R¹ und R²: unabhängig voneinander für Fluor, Chlor, Brom, Cyano, Nitro, Methyl, Ethyl, n- oder i-Propyl, n-, i-, s- oder t-Butyl, Trifluormethyl, Trichlormethyl, Trifluorethyl, Cyclopropyl, Methoxy, Ethoxy, Difluormethoxy, Trifluor- methoxy, Difluorchlormethoxy, Trifluorethoxy, Methylthio, Ethylthio, n- oder i-Propylthio, Difluormethylthio, Difluorchlormethylthio, Trifluormethylthio stehen und
- R³: für Fluor steht.

Außerdem bevorzugt sind Verbindungen der Formel (Id), 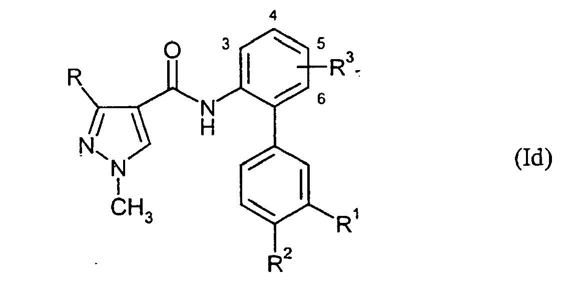 in welcher
- R: für Difluormethyl oder Trifluormethyl steht,
- R¹ und R²: unabhängig voneinander für Fluor, Chlor, Brom, Cyano, Nitro, Methyl, Ethyl, n- oder i-Propyl, n-, i-, s- oder t-Butyl, Trifluormethyl, Trichlormethyl, Trifluorethyl, Cyclopropyl, Methoxy, Ethoxy, Difluormethoxy, Trifluor- methoxy, Difluorchlormethoxy, Trifluorethoxy, Methylthio, Ethylthio, n-oder i-Propylthio, Difluormethylthio, Difluorchlotmethylthio, Trifluormethylthio stehen und
- R³ für: Fluor steht.

Außerdem bevorzugt sind Verbindungen der Formel (Ie), 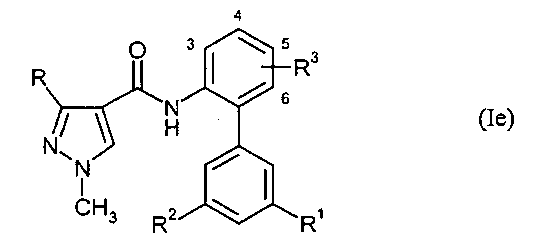 in welcher
- R: für Difluormethyl oder Trifluormethyl steht,
- R¹ und R²: unabhängig voneinander für Fluor, Chlor, Brom, Cyano, Nitro, Methyl, Ethyl, n- oder 1-Propyl, n-, i-, s- oder t-Butyl, Trifluormethyl, Trichlormethyl, Trifluorethyl, Cyclopropyl, Methoxy, Ethoxy, Difluormethoxy, Trifluor- methoxy, Difluorchlormethoxy, Trifluorethoxy, Methylthio, Ethylthio, n- oder i-Propylthio, Difluormethylthio, Difluorchlormethylthio, Trifluormethylthio stehen und
- R³ für: Fluor steht.

Die vorliegende Anmeldung betrifft insbesondere Verbindungen der Formel (Ic), in welcher
- R: für Difluormethyl oder Trifluormethyl steht,
- R¹ und R²: unabhängig voneinander für Fluor, Chlor, Brom, Methyl, Trifluormethyl, Difluormethoxy oder Trifluormethoxy stehen und
- R³: für Fluor steht.

Die vorliegende Anmeldung betrifft ebenso insbesondere Verbindungen der Formel (Id), in welcher
- R: für Difluormethyl oder Trifluormethyl steht,
- R¹ und R²: gleich oder verschieden sind und unabhängig voneinander für Fluor, Chlor, Brom, Methyl, Trifluormethyl, Difluormethoxy oder Trifluormethoxy stehen und
- R³: für Fluor steht.

Die vorliegende Anmeldung betrifft ebenso insbesondere Verbindungen der Formel (Id), in welcher
- R: für Difluormethyl oder Trifluormethyl steht,
- R¹ und R²: gleich oder verschieden sind und unabhängig voneinander für Fluor, Chlor, Brom, Methyl, Trifluormethyl, Difluormethoxy oder Trifluormethoxy stehen und
- R³: für Fluor steht.

Die vorliegende Anmeldung betrifft ganz besonders bevorzugt Verbindungen der Formel (Ie), in welcher
- R: für Difluormethyl oder Trifluormethyl steht,
- R¹ und R²: unabhängig voneinander für Fluor, Chlor, Brom, Methyl, Trifluormethyl, Difluormethoxy oder Trifluormethoxy stehen und
- R³: für 3-Fluor oder 5-Fluor steht.

Die vorliegende Anmeldung betrifft ebenso ganz besonders bevorzugt Verbindungen der Formel (Id), in welcher
- R: für Difluormethyl oder Trifluormethyl steht,
- R¹ und R²: unabhängig voneinander für Fluor, Chlor, Brom, Methyl, Trifluormethyl, Difluormethoxy oder Trifluormethoxy stehen und
- R³: für 3-Fluor oder 5-Fluor steht.

Die vorliegende Anmeldung betrifft ebenso ganz besonders bevorzugt Verbindungen der Formel (Ie), in welcher
- R: für Difluormethyl oder Trifluormethyl steht,
- R¹ und R²: unabhängig voneinander für Fluor, Chlor, Brom, Methyl, Trifluormethyl, Difluormethoxy oder Trifluormethoxy stehen und
- R³: für 3-Fluor oder 5-Fluor steht.

Die oben aufgeführten allgemeinen oder in Vorzugsbereichen aufgeführten Restedefinitionen bzw. Erläuterungen können auch untereinander, also zwischen den jeweiligen Bereichen und Vorzugsbereichen beliebig kombiniert werden. Sie gelten für die Endprodukte sowie für die Vor- und Zwischenprodukte entsprechend. Außerdem können auch einzelne Definitionen entfallen.

Gesättigte Kohlenwasserstoffreste wie Alkyl können, auch in Verbindung mit Heteroatomen, wie z.B. in Alkoxy, soweit möglich, jeweils geradkettig oder verzweigt sein.

Durch Halogen substituierte Reste, z.B. Halogenalkyl, sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Verwendet man beispielsweise 1-Methyl-3-(trifluormethyl)-1H-pyrazol-4-carbonylchlorid und 3'-Chlor-4',5-difluor-1,1'-biphenyl-2-amin als Ausgangsstoffe sowie ein Base, so kann der Verlauf des erfindungsgemäßen Verfahrens a) durch folgende Reaktionsgleichung veranschaulicht werden:

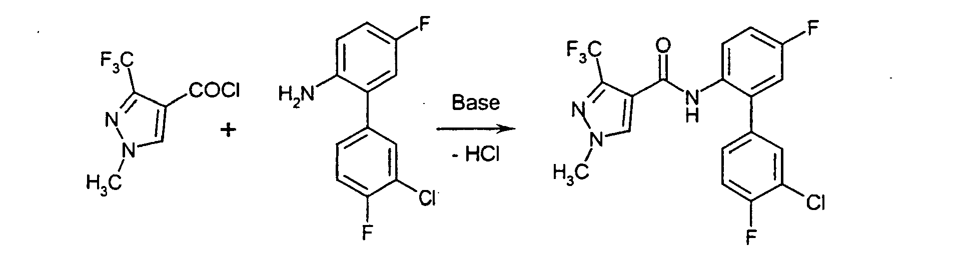

Die zur Durchführung des erfindungsgemäßen Verfahrens a) als Ausgangsstoffe benötigten Pyrazolylcarbonsäurehalogenide sind durch die Formel (II) allgemein definiert. In dieser Formel (II) steht R für Difluormethyl oder Trifluormethyl. X¹ steht bevorzugt für Chlor.

Die Pyrazolylcarbonsäurehalogenide der Formel (II) sind bekannt und/oder lassen sich nach bekannten Verfahren herstellen (vgl. z.B. JP 01290662 und US 5,093,347). Die weiterhin zur Durchführung des erfindungsgemäßen Verfahrens a) als Ausgangsstoffe benötigten Anilin-Derivate sind durch die Formel (III) allgemein definiert. In dieser Formel (III) stehen R¹, R² und R³ bevorzugt bzw. besonders bevorzugt für diejenigen Bedeutungen, die bereits im Zusammenhang mit der Beschreibung der erfindungsgemäßen Verbindungen der Formel (I) als bevorzugt bzw. besonders bevorzugt für diese Reste angegeben wurden.

Die Anilin-Derivate der Formel (III) sind noch nicht bekannt und als neue chemische Verbindungen ebenfalls Gegenstand der vorliegenden Anmeldung. Sie werden erhalten, indem man
d) Fluorhalogenaniline der Formel (VIII) 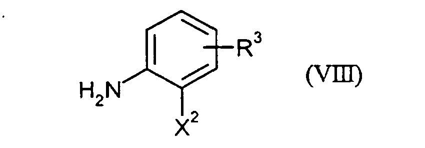 in welcher
   - R³ und X²: die oben angegebenen Bedeutungen haben,
   mit einem Boronsäurederivat der Formel (V) 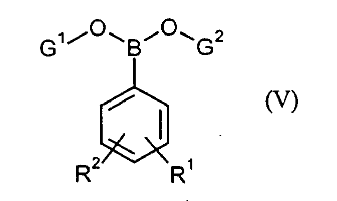 in welcher
   - R¹ und R²: die oben angegebenen Bedeutungen haben,
   - G¹ und G²: jeweils für Wasserstoff oder zusammen für Tetramethylethylen stehen,
   in Gegenwart eines Katalysators, gegebenenfalls in Gegenwart eines Säurebindemittels und gegebenenfalls in Gegenwart eines Verdünnungsmittels umsetzt.

Verwendet man beispielsweise 2-Brom-4-fluor-anilin und 3-Chlor-4-fluorphenylboronsäure als Ausgangsstoffe sowie eine Base, so kann der Verlauf des erfindungsgemäßen Verfahrens d) durch folgende Reaktionsgleichung veranschaulicht werden: 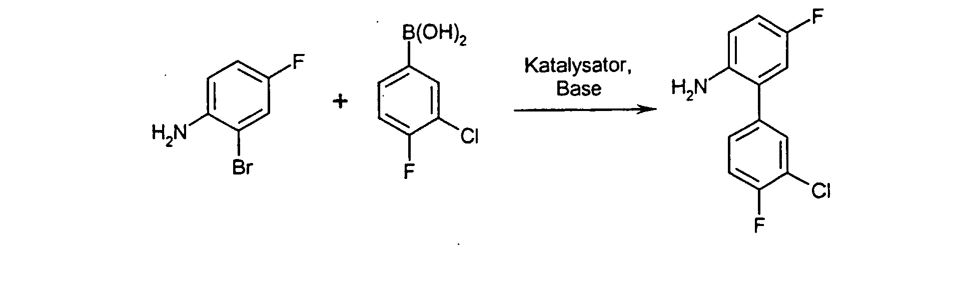 Die zur Durchführung des erfindungsgemäßen Verfahrens d) als Ausgangsstoffe benötigten Fluorhalogenaniline sind durch die Formel (VIII) allgemein definiert. In dieser Formel (VIII) steht R³ für Fluor und X² für Brom oder Iod.

Die Fluorhalogenaniline der Formel (VIII) sind bekannt oder können nach bekannten Methoden erhalten werden (vgl. z.B. US 28939 oder J. Org. Chem. 2001, 66, 4525-4542).

Die weiterhin zur Durchführung des erfindungsgemäßen Verfahrens d) als Ausgangsstoffe benötigten Boronsäurederivate sind durch die Formel (V) allgemein definiert. In dieser Formel (V) stehen R¹ und R² bevorzugt bzw. besonders bevorzugt für diejenigen Bedeutungen, die bereits im Zusammenhang mit der Beschreibung der erfindungsgemäßen Verbindungen der Formel (I) als bevorzugt bzw. besonders bevorzugt für R¹ und R² angegeben wurden. G¹ und G² stehen bevorzugt jeweils für Wasserstoff oder zusammen für Tetramethylethylen.

Boronsäuren der Formel (V) sind bekannte Synthesechemikalien. Sie können auch unmittelbar vor der Reaktion direkt aus Halogenbenzolderivaten und Boronsäureestem hergestellt und ohne Aufarbeitung weiter umgesetzt werden.

Verwendet man *N*-(2-Brom-4-fluorphenyl)-1-methyl-3-(trifluormethyl)-1H-pyrazol-4-carboxamid und 3-Chlor-4-fluorphenylboronsäure als Ausgangsstoffe sowie einen Katalysator und eine Base, so kann der Verlauf des erfindungsgemäßen Verfahrens b) durch folgende Reaktionsgleichung veranschaulicht werden:

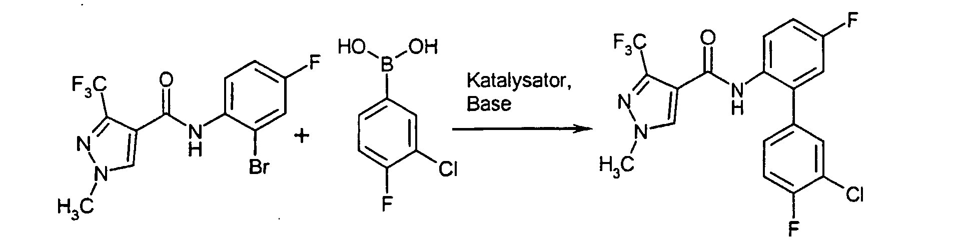

Die zur Durchführung des erfindungsgemäßen Verfahrens b) als Ausgangsstoffe benötigten Halogenpyrazolcarboxanilide sind durch die Formel (IV) allgemein definiert. In dieser Formel (IV) stehen R und R³ bevorzugt bzw. besonders bevorzugt für diejenigen Bedeutungen, die bereits im Zusammenhang mit der Beschreibung der erfindungsgemäßen Verbindungen der Formel (I) als bevorzugt bzw. besonders bevorzugt für diese Reste angegeben wurden. X² steht bevorzugt für Brom oder Iod. Die Halogenpyrazolcarboxanilide der Formel (IV) sind noch nicht bekannt. Sie sind neue chemische Verbindungen und ebenfalls Gegenstand der vorliegenden Anmeldung. Sie werden erhalten, indem man
e) Pyrazolylcarbonsäurehalogenide der Formel (II) 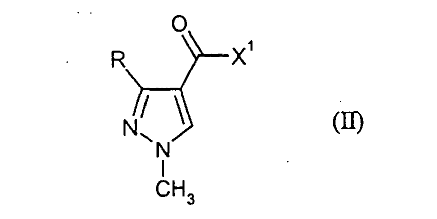 in welcher
   - R: die oben angegebenen Bedeutungen hat,
   - X¹: für Halogen steht,
   mit Fluorhalogenanilinen der Formel (VIII) 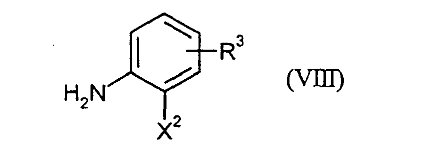 in welcher
   - R³ und X²: die oben angegebenen Bedeutungen haben
   gegebenenfalls in Gegenwart eines Säurebindemittels und gegebenenfalls in Gegenwart eines Verdünnungsmittels umsetzt.

Verwendet man beispielsweise 1-Methyl-3-(trifluormethyl)-1H-pyrazol-4-carbonylchlorid und 2-Brom-4-fluoranilin als Ausgangsstoffe sowie ein Base, so kann der Verlauf des erfindungsgemäßen Verfahrens e) durch folgende Reaktionsgleichung veranschaulicht werden:

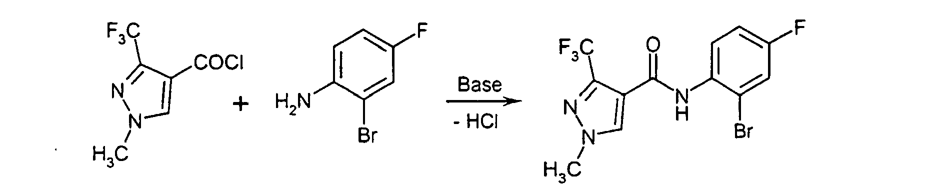

Die zur Durchführung des erfindungsgemäßen Verfahrens e) als Ausgangsstoffe benötigten Pyrazolylcarbonsäurehalogenide der Formel (II) sind bereits weiter oben im Zusammenhang mit dem erfindungsgemäßen Verfahren a) beschrieben worden.

Die weiterhin zur Durchführung des erfindungsgemäßen Verfahrens e) als Ausgangsstoffe benötigten Fluorhalogenaniline der Formel (VIII) sind bereits weiter oben im Zusammenhang mit dem erfindungsgemäßen Verfahren d) beschrieben worden.

Die weiterhin zur Durchführung des erfindungsgemäßen Verfahrens b) als Ausgangsstoffe benötigten Boronsäuren der Formel (V) sind bereits weiter oben im Zusammenhang mit dem erfindungsgemäßen Verfahren d) beschrieben worden.

Verwendet man beispielsweise *N*-(2-Brom-4-fluorphenyl)-1-methyl-3-(trifluormethyl)-1H-pyrazol-4-carboxamid und 4,4,4',4',5,5,5',5'-Octamethyl-2,2'-bi-1,3,2-dioxaborolan in der ersten Stufe und weiterhin 4-Brom-2-chlor-1-fluorbenzol in der zweiten Stufe als Ausgangsstoffe, sowie in jeder Stufe einen Katalysator und eine Base, so kann der Verlauf des erfindungsgemäßen Verfahrens c) durch folgende Reaktionsgleichung veranschaulicht werden:

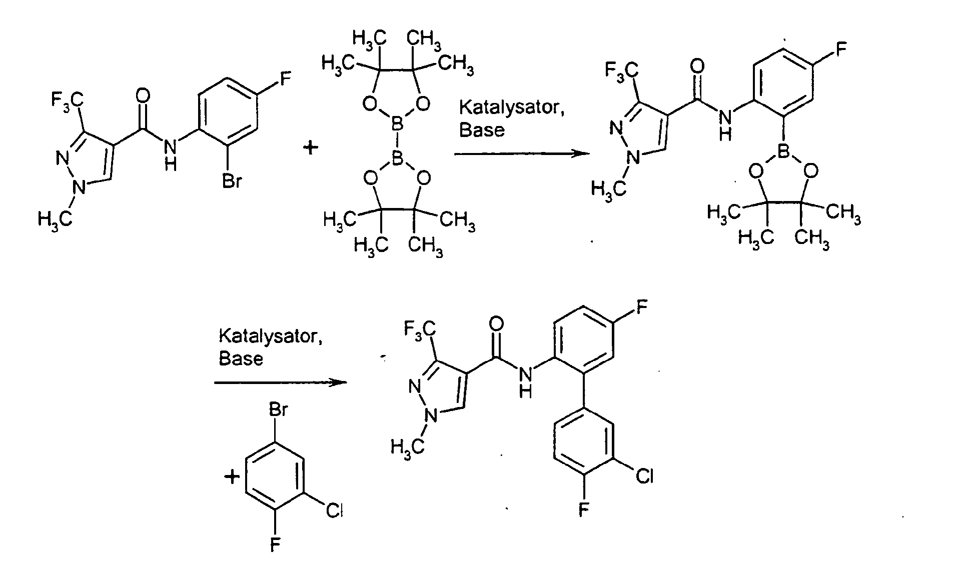

Die zur Durchführung des erfindungsgemäßen Verfahrens c) als Ausgangsstoffe benötigten Halogenpyrazolcarboxanilide der Formel (IV) sind bereits weiter oben im Zusammenhang mit dem erfindungsgemäßen Verfahren b) beschrieben worden.

Die zur Durchführung des erfindungsgemäßen Verfahrens c) weiterhin als Ausgangsstoffe benötigten Diboran-Derivate sind durch die Formel (VI) allgemein definiert. In dieser Formel (VI) stehen G³ und G⁴ bevorzugt jeweils für Methyl, Ethyl, Propyl, Butyl oder gemeinsam für Tetramethylethylen.

Die Diboran-Derivate der Formel (VI) sind allgemein bekannte Synthesechemikalien.

Die außerdem zur Durchführung des erfindungsgemäßen Verfahrens c) als Ausgangsstoffe benötigten Halogenbenzolderivate sind durch die Formel (VII) allgemein definiert. In dieser Formel (VII) stehen R¹ und R² bevorzugt bzw. besonders bevorzugt für diejenigen Bedeutungen, die bereits im Zusammenhang mit der Beschreibung der erfindungsgemäßen Verbindungen der Formel (I) als bevorzugt bzw. besonders bevorzugt für diese Reste angegeben wurden. X³ steht für bevorzugt für Brom, Iod oder Trifluormethylsulfonyloxy.

Die Halogenbenzolderivate der Formel (VII) sind allgemein bekannte Synthesechemikalien.

Als Verdünnungsmittel zur Durchführung der erfindungsgemäßen Verfahrens a) und e) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Die erfindungsgemäßen Verfahren a) und e) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Caesiumcarbonat, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren a) und e) in einem größeren Bereich variiert werden. Im allgemeinen arbeitet man bei Temperaturen von 0°C bis 150°C, vorzugsweise bei Temperaturen von 20°C bis 110°C.

Zur Durchführung des erfindungsgemäßen Verfahrens a) zur Herstellung der Verbindungen der Formel (I) setzt man pro Mol des Pyrazolylcarbonsäurehalogenides der Formel (II) im allgemeinen 0,2 bis 5 Mol, vorzugsweise 0,5 bis 2 Mol an Anilinderivat der Formel (III) ein.

Zur Durchführung des erfindungsgemäßen Verfahrens e) zur Herstellung der Verbindungen der Formel (IV) setzt man pro Mol des Pyrazolylcarbonsäurehalogenides der Formel (II) im allgemeinen 0,2 bis 5 Mol, vorzugsweise 0,5 bis 2 Mol an Fluorhalogenanilin der Formel (VIII) ein.

Als Verdünnungsmittel zur Durchführung der erfindungsgemäßen Verfahren b), c) und d) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, s- oder t-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Die Reaktionstemperaturen können bei der Durchführung der erfindungsgemäßen Verfahrens b), c) und d) in einem größeren Bereich variiert werden. Im allgemeinen arbeitet man bei Temperaturen von 0°C bis 150°C, vorzugsweise bei Temperaturen von 20°C bis 110°C.

Die erfindungsgemäßen Verfahren b), c) werden und d) gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -fluoride, -phosphate, -carbonate oder -hydrogencarbonate, wie beispielsweise Natriumhydrid, Natriumamid, Lithiumdiisopropylamid, Natriummethylat, Natriumethylat, Kalium-tert.-butylat, Natriumhydroxid, Kaliumhydroxid, Natriumacetat, Kaliumacetat, Natriumphosphat, Kaliumphosphat, Kaliumfluorid, Cäsiumfluorid, Natriumcarbonat, Kaliumcarbonat, Kaliumhydrogencarbonat, Natriumhydrogencarbonat oder Cäsiumcarbonat, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die erfindungsgemäßen Verfahren b), c) und d) werden in Gegenwart eines Katalysators, wie beispielsweise eines Palladiumsalzes oder -komplexes, durchgeführt. Hierzu kommen vorzugsweise Palladiumchlorid, Palladiumacetat, Tetrakis-(triphenylphosphin)-palladium, Bis-(triphenylphosphin)-palladiumdichlorid oder 1,1'-Bis-(diphenylphosphino)ferrocenpalladium(II)chlorid infrage.

Es kann auch ein Palladiumkomplex in der Reaktionsmischung erzeugt werden, wenn man ein Palladiumsalz und einen Komplexligand, wie beispielsweise Triethylphosphan, Tri-tert-butylphosphan, Tricyclohexylphosphan, 2-(Dicyclohexylphosphan)-biphenyl, 2-(Di-tert-butylphosphan)-biphenyl, 2-(Dicyclohexylphosphan)-2'-(N,N-dimethylamino)-biphenyl, Triphenylphosphan, Tris-(o-tolyl)-phosphan, Natrium-3-(diphenylphosphino)benzolsulfonat, Tris-2-(methoxyphenyl)-phosphan, 2,2'-Bis-(diphenylphosphan)-1,1'-binaphthyl, 1,4-Bis(diphenylphosphan)-butan, 1,2-Bis-(diphenylphosphan)-ethan, 1,4-Bis(dicyclohexylphosphan)-butan, 1,2-Bis-(dicyclohexylphosphan)-ethan, 2-(Dicyclohexylphosphan)-2'-(N,N-dimethylamino)-biphenyl, Bis(diphenylphosphino)ferrocen oder Tris-(2,4-tert-butylphenyl)-phosphit getrennt zur Reaktion zugibt.

Zur Durchführung des erfindungsgemäßen Verfahrens b) zur Herstellung der Verbindungen der Formel (I) setzt man pro Mol des Halogenpyrazolcarboxanilids der

Formel (IV) im allgemeinen 1 bis 15 Mol, vorzugsweise 1 bis 5 Mol an Boronsäurederivat der Formel (V) ein.

Zur Durchführung des erfindungsgemäßen Verfahrens c) zur Herstellung der Verbindungen der Formel (I) setzt man pro Mol des Halogenpyrazolcarboxanilids der Formel (IV) im allgemeinen 1 bis 15 Mol, vorzugsweise 1 bis 5 Mol an Diboran-Derivat der Formel (VI) und 1 bis 15 Mol, vorzugsweise 1 bis 5 Mol an Halogenbenzolderivat der Formel (VII) ein.

Zur Durchführung des erfindungsgemäßen Verfahrens d) zur Herstellung der Verbindungen der Formel (III) setzt man pro Mol des Fluorhalogenanilins der Formel (VIII) im allgemeinen 1 bis 15 Mol, vorzugsweise 1 bis 5 Mol an Boronsäurederivat der Formel (V) ein.

Die erfindungsgemäßen Verfahren a), b), c), d) und e) werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Anen, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe eignen sich auch zur Steigerung des Emteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Altemaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose. Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

2-Phenylphenol; 8-Hydroxychinolinsulfat;
Acibenzolar-S-methyl; Aldimorph; Amidoflumet; Ampropylfos; Ampropylfos-potassium; Andoprim; Anilazine; Azaconazole; Azoxystrobin;
Benalaxyl; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamin;
Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram;
Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon;
Edifenphos; Epoxiconazole; Ethaboxam; Ethirimol; Etridiazole;
Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox;
Guazatine; Hexachlorobenzene; Hexaconazole; Hymexazol;
Imazalil; Imibenconazole; Iminoctadine triacetate; Iminoctadine tris(albesil); Iodocarb; Ipconazole; Iprobenfos; Iprodione; Iprovalicarb; Irumamycin; Isoprothiolane; Isovaledione; Kasugamycin; Kresoxim-methyl;
Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin;
Natamycin; Nicobifen; Nitrothal-isopropyl; Noviflumuron; Nuarimol;
Ofurace; Orysastrobin; Oxadixyl; Oxolinic acid; Oxpoconazole; Oxycarboxin; Oxyfenthiin;
Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine;
Quinconazole; Quinoxyfen; Quintozene; Simeconazole; Spiroxamine; Sulfur;
Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-Chlorphenyl)-2-propinyl]oxy]-3-methoxyphenyl]ethyl]-3-methyl- 2-[(methylsulfonyl)amino]-butanamid;
1-(1-Naphthalenyl)-1H-pyrrol-2,5-dion;
2,3,5,6-Tetrachlor-4-(methylsulfonyl)-pyridin;
2-Amino-4-methyl-N-phenyl-5-thiazolcarboxamid;
2-Chlor-N-(2,3-dihydro-1,1,3-trimethyl-1H-inden-4-yl)-3-pyridincarboxamide; 3,4,5-Trichlor-2,6-pyridindicarbonitril;
Actinovate; cis-1-(4-Chlorphenyl)-2-(1H-1,2,4-triazol-1-yl)-cycloheptanol;
Methyl 1-(2,3-dihydro-2,2-dimethyl-1H-inden-1-yl)-1H-imidazol-5-carboxylat; Monokaliumcarbonat;
N-(6-Methoxy-3-pyridinyl)-cyclopropancarboxamid;
N-Butyl-8-(1,1-dimethylethyl)-1-oxaspiro[4.5]decan-3-amin; Natriumtetrathiocarbonat;
sowie Kupfersalze und -zubereitungen, wie Bordeaux mixture; Kupferhydroxid; Kupfernaphthenat; Kupferoxychlorid; Kupfersulfat; Cufraneb; Kupferoxid; Mancopper; Oxine-copper.

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Allethrin 1R-isomers, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin,
Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben,
Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifos-methyl, Chlorpyrifos (-ethyl), Chlovaporthrin, Chromafenozide, Cis-Cypermethrin, Cis-Resmethrin, Cis-Permethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-trans-isomer), Cyromazine,
DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimethoate, Dimethylvinphos, Dinobuton, Dinocap, Dinotefuran, Diofenolan, Disulfoton, Docusat-sodium, Dofenapyn, DOWCO-439,
Eflusilanate, Emamectin, Emamectin-benzoate, Empenthrin (1R-isomer), Endosulfan, Entomopthora spp., EPN, Esfenvalerate, Ethiofencarb, Ethiprole, Ethion, Ethoprophos, Etofenprox, Etoxazole, Etrimfos,
Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb,
Gamma-HCH, Gossyplure, Grandlure, Granuloseviren,
Halfenprox, Halofenozide, HCH, HCN-801, Heptenophos, Hexaflumuron, Hexythiazox, Hydramethylnone, Hydroprene,
IKA-2002, Imidacloprid, Imiprothrin, Indoxacarb, Iodofenphos, Iprobenfos, Isazofos, Isofenphos, Isoprocarb, Isoxathion, Ivermectin,
Japonilure, Kadethrin, Kempolyederviren, Kinoprene,
Lambda-Cyhalothrin, Lindane, Lufenuron,
Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metolcarb, Metoxadiazone, Mevinphos, Milbemectin, Milbemycin, MKI-245, MON-45700, Monocrotophos, Moxidectin, MTI-800,
Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron,
OK-5101, OK-5201, OK-9601, OK-9602, OK-9701, OK-9802, Omethoate, Oxamyl, Oxydemeton-methyl,
Paecilomyces fumosoroseus, Parathion-methyl, Parathion (-ethyl), Permethrin (cis-, trans-), Petroleum, PH-6045, Phenothrin (IR-trans isomer), Phenthoate, Phorate, Phosalone, Phosmet, Phosphamidon, Phosphocarb, Phoxim, Piperonyl butoxide, Pirimicarb, Pirimiphos-methyl, Pirimiphos-ethyl, Prallethrin, Profenofos, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen,
Quinalphos,
Resmethrin, RH-5849, Ribavirin, RU-12457, RU-15525,
S-421, S-1833, Salithion, Sebufos, SI-0009, Silafluofen, Spinosad, Spirodiclofen, Spiromesifen, Sulfluramid, Sulfotep, Sulprofos, SZI-121,
Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (1R-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Triflumuron, Trimethacarb,
Vamidothion, Vaniliprole, Verbutin, Verticillium lecanii,
WL-108477, WL-40027, YI-5201, YI-5301, YI-5302, XMC, Xylylcarb,
ZA-3274, Zeta-Cypermethrin, Zolaprofos, ZXI-8901,
die Verbindung 3-Methyl-phenyl-propylcarbamat (Tsumacide Z),
die Verbindung 3-(5-Chlor-3-pyridinyl)-8-(2,2,2-trifluorethyl)-8-azabicyclo[3.2.1]-octan-3-carbonitril (CAS-Reg.-Nr. 185982-80-3) und das entsprechende 3-endo-Isomere (CAS-Reg.-Nr. 185984-60-5) (vgl. WO-96/37494, WO-98/25923),
sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Emährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Namatoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD^{®} (z.B. Mais, Baumwolle, Soja), KnockOut^{®} (z.B. Mais), StarLink^{®} (z.B. Mais), Bollgard^{®} (Baumwolle), Nucoton^{®} (Baumwolle) und NewLeaf^{®} (Kartoffel) vertrieben werden. Als Beispiele für Herbizid tolerante Pflanzen seien Maissorten, Baumwollsorten und Sojasorten genannt, die unter den Handelsbezeichnungen Roundup Ready^{®} (Toleranz gegen Glyphosate z.B. Mais, Baumwolle, Soja), Liberty Link^{®} (Toleranz gegen Phosphinotricin, z.B. Raps), IMI^{®} (Toleranz gegen Imidazolinone) und STS^{®} (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield^{®} vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Wirkstoffe geht aus den folgenden Beispielen hervor.

### Herstellungsbeispiele

### Beispiel 1

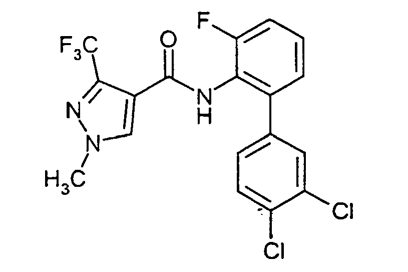

### Verfahren a)

0,333 g (1,3 mmol) 3',4'-Dichlor-3-fluor-1,1'-biphenyl-2-amin und 0,33 g (1,56 mmol) 1-Methyl-3-(trifluormethyl)-1H-pyrazol-4-carbonylchlorid werden in 6 ml Tetrahydrofuran gelöst und mit 0,36 ml (2,6 mmol) Triethylamin versetzt. Die Reaktionslösung wird 16 Stunden bei 60°C gerührt. Zur Aufarbeitung wird aufkonzentriert und mit Cyclohexan/Essigsäureethylester an Kieselgel chromatographiert.

Man erhält 0,39 g (72 % d. Th.) *N*-(3',4'-Dichlor-3-fluor-1,1'-biphenyl-2-yl)-1-methyl-3-(trifluormethyl)-1H-pyrazol-4-carboxamid mit dem logP (pH2,3) = 3.10.

### Beispiel 2

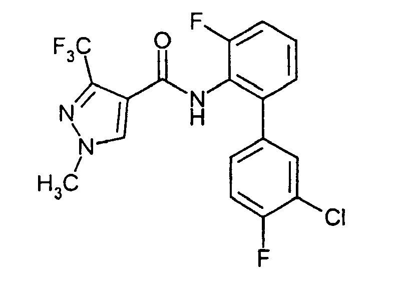

### Verfahren b)

0,256 g (0,7 mmol) N-(2-Brom-6-fluorphenyl)-1-methyl-3-(trifluormethyl)-1H-pyrazol-4-carboxamid und 0,12 g (0,77 mmol) 3-Chlor-4-fluorphenylboronsäure werden unter Ausschluss von Sauerstoff in einer Mischung aus 8 ml Toluol, 1,5 ml Ethanol und 5,25 ml gesättigter Natriumcarbonatlösung unter Argon suspendiert. Die Reaktionsmischung wird mit einer katalytischen Menge (0,01-0,3 Äquivalente) Tetrakis-(triphenylphosphin)palladium(0) versetzt und unter Argon eine Stunde auf 100°C erwärmt. Die organische Phase wird abgetrennt und die wässrige Phase mit Essigsäureethylester extrahiert. Die vereinigten organischen Phasen werden eingeengt und der Rückstand wird mit Cyclohexan/Essigsäureethylester (1:1) an Kieselgel chromatographiert.

Man erhält 0,27 g (96 % d. Th.) *N*-(3'-Chlor-3,4'-difluor-1,1'-biphenyl-2-yl)-1-methyl-3-(trifluorethyl)-1H-pyrazol-4-carboxamid mit dem logP (pH2,3) = 3.04.

Analog den Beispielen 1 und 2, sowie entsprechend den Angaben in den allgemeinen Beschreibungen der Verfahren a) und b), werden die in der nachstehenden Tabelle 1 genannten Verbindungen der Formel (I) erhalten.

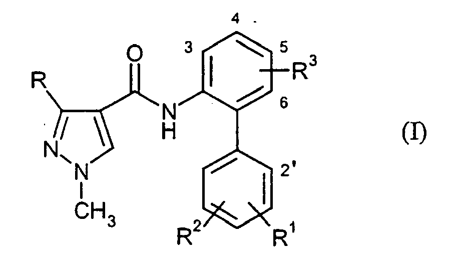

**Tabelle 1**

| **Bsp.** | **R** | **R¹** | **R²** | **R³** | **logP** |
|---|---|---|---|---|---|
| 3 | CF₃ | 3'-Cl | 4'-Cl | 4-F | 3,75 |
| 4 | CF₃ | 3'-Cl | 4'-F | 4-F | 3,51 |
| 5 | CF₃ | 3'-Cl | 4'-Cl | 5-F | 3,57 |
| 6 | CF₃ | 3'-Cl | 4'-F | 5-F | 3,26 |
| 7 | CHF₂ | 3'-Cl | 4'-Cl | 3-F | 3,10 |
| 8 | CHF₂ | 3'-Cl | 4'-F | 3-F | 2,83 |
| 9 | CHF₂ | 3'-Cl | 4'-Cl | 4-F | 3,55 |
| 10 | CHF₂ | 3'-Cl | 4'-F | 4-F | 3,29 |
| 11 | CF₂ | 3'-Cl | 4'-Cl | 5-F | 3,33 |
| 12 | CHF₂ | 3'-Cl | 4'-F | 5-F | 3,07 |
| 13 | CHF₂ | 3'-F | 5'-F | 6-F | 2,64 |
| 14 | CHF₂ | 3'-F | 5'-F | 4-F | 3,03 |
| 15 | CHF₂ | 3'-F | 5'-F | 5-F | 2,81 |

### Herstellung eines Vorproduktes der Formel (III)

### Beispiel (III-1)

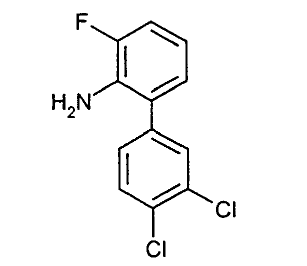

### Verfahren d)

51,2 g (0,268 mol) 3,4-Dichlorphenylboronsäure und 42,5 g (0,223 mol) 2-Brom-6-fluoranilin werden unter Ausschluss von Sauerstoff in einer Mischung aus 300 ml Toluol, 30 ml Ethanol und 220 ml gesättigter Natriumcarbonatlösung unter Argon suspendiert. Die Reaktionsmischung wird mit 2,6 g Tetrakis(triphenylphosphin)-palladium(0) versetzt und 12 Stunden bei 80°C gerührt. Die organische Phase wird abgetrennt und die wässrige Phase mit Essigsäureethylester extrahiert. Die vereinigten organischen Phasen werden eingeengt und der Rückstand wird mit Cyclohexan/Essigsäureethylester (3:1) an Kieselgel chromatographiert.

Man erhält 37,4 g (65 % d. Th.) 3',4'-Dichlor-3-fluor-1,1'-biphenyl-2-amin mit dem logP (pH2,3) = 4,09.

### Beispiel (III-2)

Analog Beispiel (III-1) wurde 3',4'-Dichlor-5-fluor-1,1'-blphenyl-2-amin mit dem logP (pH2,3) = 3,62 erhalten.

### Herstellung der Vorprodukte der Formel (IV)

### Beispiel (IV-1)

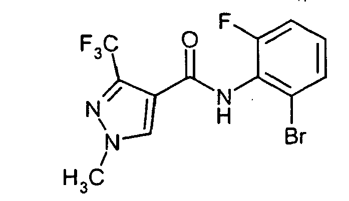

### Verfahren e)

1,0 g (5,6 mmol) 2-Brom-6-fluoranilin werden in 5 ml Toluol gelöst und mit einer Lösung von 0,6 g (2,8 mmol) 1-Methyl-3-(trifluormethyl)-1H-pyrazol-4-carbonylchlorid in 2 ml Toluol versetzt. Die Reaktionslösung wird 12 Stunden unter Rückfluss erhitzt. Zur Aufreinigung wird die Reaktionsmischung über eine kombinierte Säule bestehend aus saurem Anionentauscher und Kieselgel mit Essigsäureethylester eluiert.

Man erhält 0,57 g (55,6 % d. Th.) *N*-(2-Brom-6-fluorphenyl)-1-methyl-3-(trifluorethyl)-1H-pyrazol-4-carboxamid mit dem logP (pH2,3) = 2,12.

Analog Beispiel (IV-1) sowie entsprechend den Angaben in der allgemeinen Beschreibungen des Verfahrens e), werden die in der nachstehenden Tabelle 2 genannten Verbindungen der Formel (IV) erhalten.

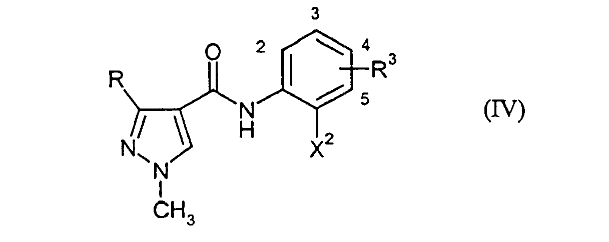

**Tabelle 2**

| **Bsp.** | **R** | **R³** | **X²** | **logP** |
|---|---|---|---|---|
| IV-2 | CF₃ | 3-F | Br | 2,80 |
| IV-3 | CF₃ | 4-F | Br | 2,52 |
| IV-4 | CHF₂ | 2-F | Br | 1,89 |
| IV-5 | CHF₂ | 3-F | Br | 2,53 |
| IV-6 | CHF₂ | 4-F | Br | 2,26 |

Die Bestimmung der in den voranstehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung erfolgt im sauren Bereich bei pH 2.3 mit 0,1 % wässriger Phosphorsäure und Acetonitril als Eluenten; linearer Gradient von 10 % Acetonitril bis 90 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele

### Beispiel A

### Podosphaera-Test (Apfel) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension des Apfelmehltauerregers *Podosphaera leucotricha* inokuliert. Die Pflanzen werden dann im Gewächshaus bei ca. 23°C und einer relativen Luftfeuchtigkeit von ca. 70 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle A**

| **Podosphaera-Test (Apfel) / protektiv** | | | |
|---|---|---|---|
| Wirkstoff | | Aufwandmenge an Wirkstoff in g/ha | Wirkungsgrad in % |
| 1 | 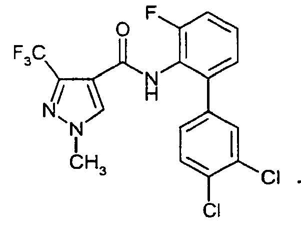 | 100 | 100 |
| 3 | 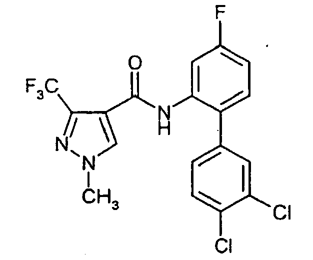 | 100 | 100 |
| 4 | 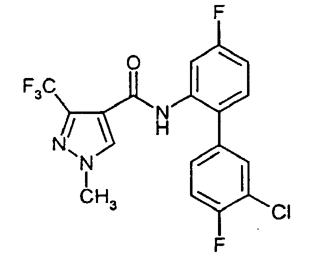 | 100 | 100 |
| 5 | 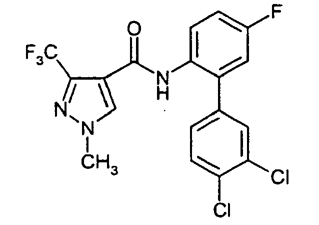 | 100 | 100 |
| 6 | 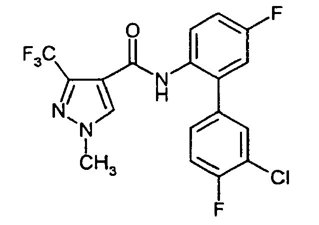 | 100 | 100 |
| 7 | 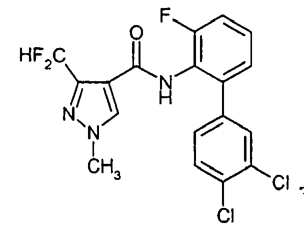 | 100 | 100 |
| 9 | 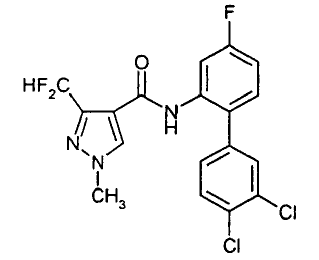 | 100 | 83 |
| 10 | 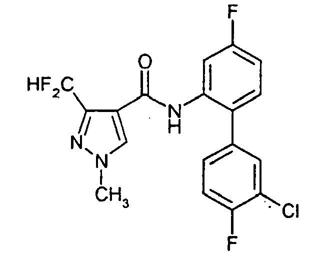 | 100 | 100 |
| 11 | 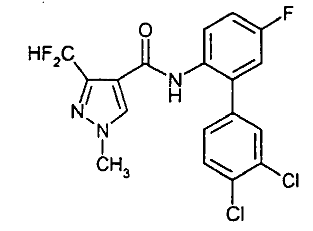 | 100 | 100 |
| 12 | 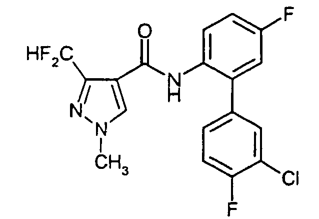 | 100 | 100 |

### Beispiel B

### Sphaerotheca-Test (Gurke) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von *Sphaerotheca fuliginea* inokuliert. Die Pflanzen werden dann bei ca. 23°C und einer relativen Luftfeuchtigkeit von ca. 70 % im Gewächshaus aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle B**

| **Sphaerotheca-Test (Gurke) / protektiv** | | | |
|---|---|---|---|
| Wirkstoff | | Aufwandmenge an Wirkstoff in g/ha | Wirkungsgrad in % |
| 1 | 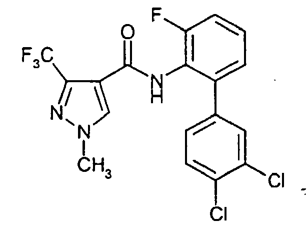 | 100 | 100 |
| 3 | 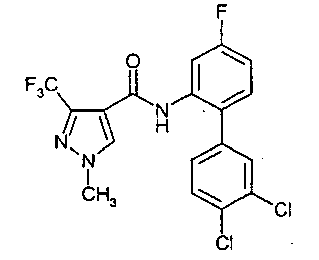 | 100 | 100 |
| 4 | 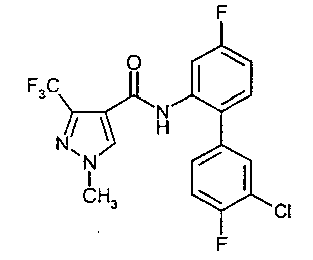 | 100 | 99 |
| 5 | 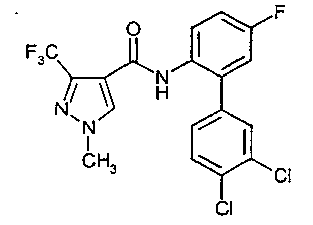 | 100 | 100 |
| 6 | 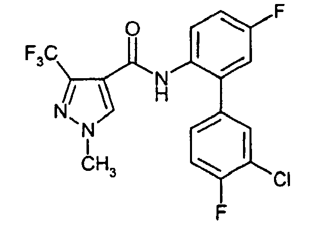 | 100 | 98 |
| 7 | 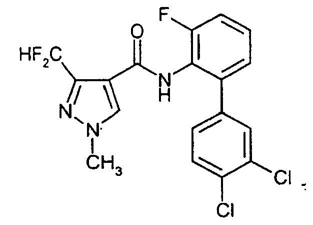 | 100 | 100 |
| 9 | 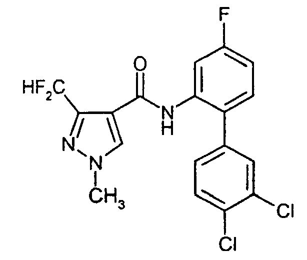 | 100 | 94 |
| 10 | 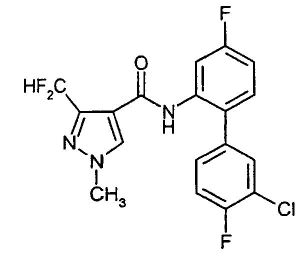 | 100 | 100 |
| 11 | 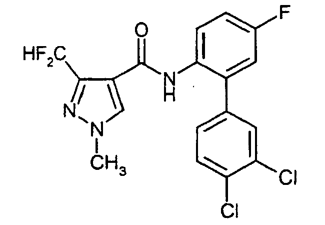 | 100 | 100 |
| 12 | 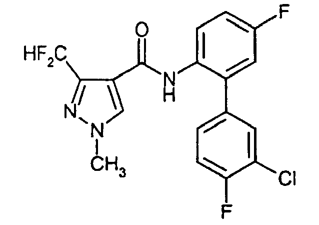 | 100 | 100 |

### B eispiel C

### Venturia - Test (Apfel) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers *Venturia inaequalis* inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle C**

| **Venturia - Test (Apfel) / protektiv** | | | |
|---|---|---|---|
| Wirkstoff | | Aufwandmenge an Wirkstoff in g/ha | Wirkungsgrad in % |
| 1 | 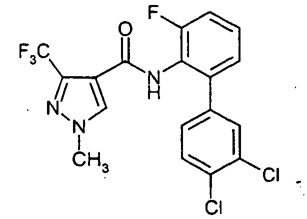 | 100 | 100 |
| 3 | 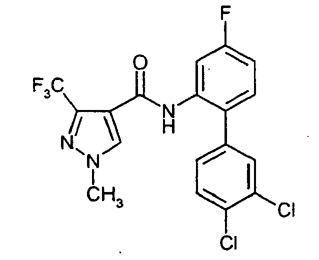 | 100 | 100 |
| 4 | 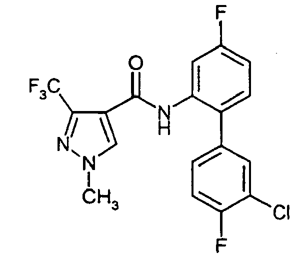 | 100 | 100 |
| 5 | 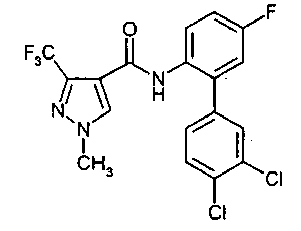 | 100 | 100 |
| 6 | 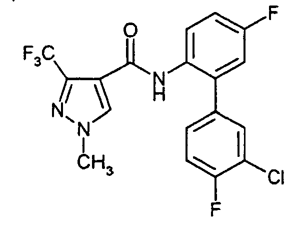 | 100 | 100 |
| 7 | 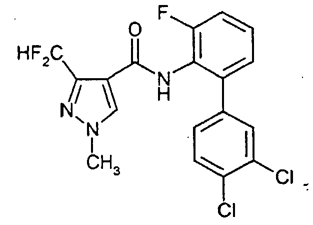 | 100 | 100 |
| 9 | 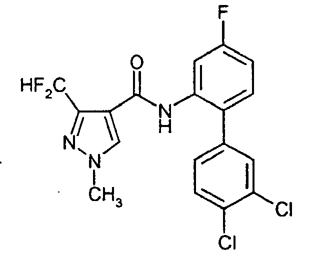 | 100 | 99 |
| 10 | 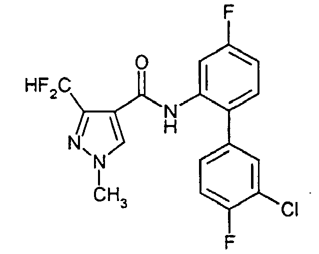 | 100 | 100 |
| 11 | 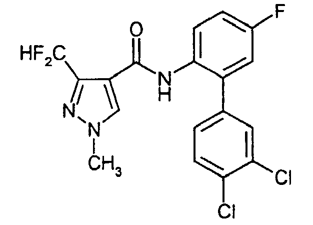 | 100 | 100 |
| 12 | 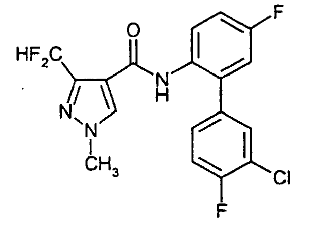 | 100 | 100 |

### Beispiel D

### Alternaria-Test (Tomate) / protektiv

| | |
|---|---|
| Lösungsmittel: | 49 Gewichtsteile N,N-Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer Sporensuspension von *Alternaria solani* inokuliert und stehen dann 24h bei 100% rel. Feuchte und 20°C. Anschließend stehen die Pflanzen bei 96 % relativer Luftfeuchtigkeit und einer Temperatur von 20°C.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle D**

| **Alternaria-Test (Tomate) / protektiv** | | | |
|---|---|---|---|
| Wirkstoff | | Aufwandmenge an Wirkstoff in g/ha | Wirkungsgrad in % |
| 1 | 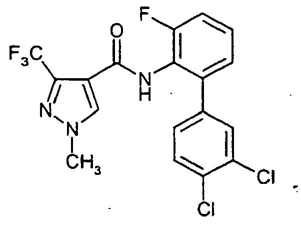 | 750 | 100 |
| 2 | 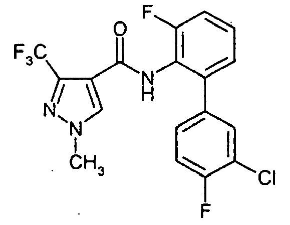 | 750 | 100 |
| 3 | 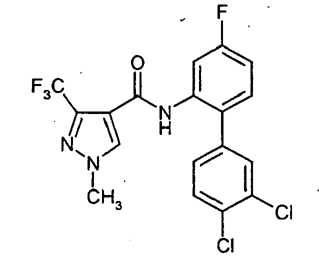 | 750 | 100 |
| 4 | 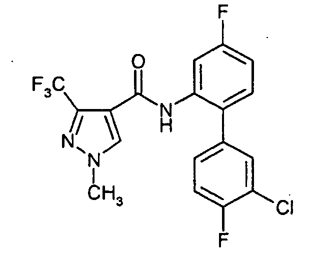 | 750 | 100 |
| 5 | 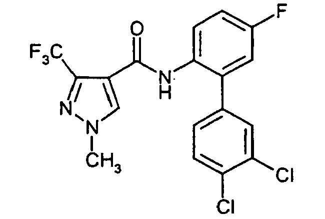 | 750 | 100 |
| 6 | 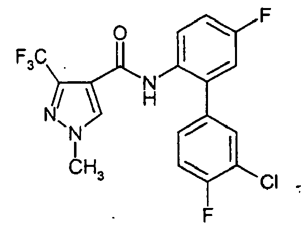 | 750 | 100 |

## Patentansprüche

1. Pyrazolylcarboxanilide der Formel (I) 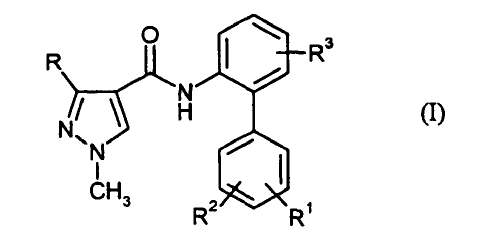 in welcher
R für Difluormethyl oder Trifluormethyl steht,
R¹ und R² unabhängig voneinander für Halogen, Cyano, Nitro, C₁-C₆-Alkyl, C₂-C₆-Alkenyl, C₁-C₄-Alkoxy, C₁-C₄-Alkylthio, C₁-C₄-Alkylsulfonyl, C₃-C₆-Cycloalkyl, oder für C₁-C₄-Halogenalkyl, C₁-C₄-Halogen- alkoxy, C₁-C₄-Halogenalkylthio oder C₁-C₄-Halogenalkylsulfonyl mit jeweils 1 bis 5 Halogenatomen stehen,
R³ für Fluor steht.

2. Pyrazolylcarboxanilide der Formel (I) gemäß Anspruch 1, in welcher
R für Difluormethyl oder Trifluormethyl steht,
R¹ und R² unabhängig voneinander für Fluor, Chlor, Brom, Cyano, Nitro, Methyl, Ethyl, n- oder i-Propyl, n-, i-, s- oder t-Butyl, Methoxy, Ethoxy, Methylthio, Ethylthio, n- oder i-Propylthio, Cyclopropyl, Tri- fluormethyl, Trichlormethyl, Trifluorethyl, Difluormethoxy, Trifluor- methoxy, Difluorchlormethoxy, Trifluorethoxy, Difluornethylthio, Difluorchlormethylthio oder Trifluormethylthio stehen,
R³ für Fluor steht.

3. Pyrazolylcarboxanilide der Formel (I) gemäß Anspruch 1, in welcher
R für Difluormethyl oder Trifluormethyl steht,
R¹ und R² unabhängig voneinander für Fluor, Chlor, Brom, Methyl, Trifluor- methyl, Difluormethoxy oder Trifluormethoxy stehen,
R³ für Fluor steht.

4. Pyrazolylcarboxanilide der Formel (I) gemäß Anspruch 1, in welcher R¹ für Fluor und R² für Chlor steht.

5. Pyrazolylcarboxanilide der Formel (I) gemäß Anspruch 1, in welcher R¹ für Fluor und R² für Fluor steht.

6. Pyrazolylcarboxanilide der Formel (I) gemäß Anspruch 1, in welcher R¹ für Methyl oder Trifluormethyl steht.

7. Verbindungen der Formel (Ia), 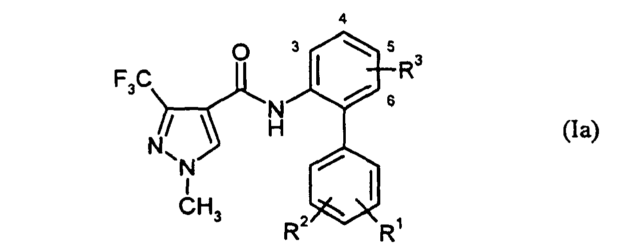 in welcher
R¹ und R² unabhängig voneinander für Fluor, Chlor, Brom, Cyano, Nitro, Methyl, Ethyl, n- oder i-Propyl, n-, i-, s- oder t-Butyl, Trifluormethyl, Trichlormethyl, Trifluorethyl, Cyclopropyl, Methoxy, Ethoxy, Difluor- methoxy, Trifluormethoxy, Difluorchlormethoxy, Trifluorethoxy, Methylthio, Ethylthio, n- oder i-Propylthio, Difluormethylthio, Di- fluorchlormethylthio, Trifluormethylthio stehen und
R³ für Fluor steht.

8. Verbindungen der Formel (Ib), 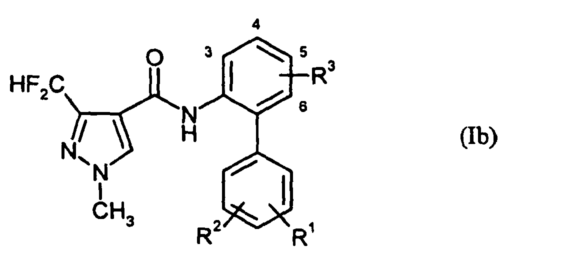 in welcher
R¹ und R² unabhängig voneinander für Fluor, Chlor, Brom, Cyano, Nitro, Methyl, Ethyl, n- oder i-Propyl, n-, i-, s- oder t-Butyl, Trifluormethyl, Trichlormethyl, Trifluorethyl, Cyclopropyl, Methoxy, Ethoxy, Difluor- methoxy, Trifluormethoxy, Difluorchlormethoxy, Trifluorethoxy, Me- thylthio, Ethylthio, n- oder i-Propylthio, Difluormethylthio, Difluor- chlormethylthio, Trifluormethylthio stehen und
R³ für Fluor steht.

9. Verbindungen der Formel (Ic), 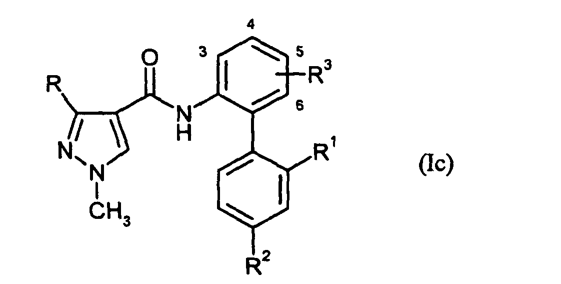 in welcher
R für Difluormethyl oder Trifluormethyl steht,
R¹ und R² unabhängig voneinander für Fluor, Chlor, Brom, Cyano, Nitro, Methyl, Ethyl, n- oder i-Propyl, n-, i-, s- oder t-Butyl, Trifluormethyl, Trichlormethyl, Trifluorethyl, Cyclopropyl, Methoxy, Ethoxy, Difluor- methoxy, Trifluormethoxy, Difluorchlormethoxy, Trifluorethoxy, Me- thylthio, Ethylthio, n- oder i-Propylthio, Difluormethylthio, Difluor- chlormethylthio, Trifluormethylthio stehen und
R³ für Fluor steht.

10. Verbindungen der Formel (Id), 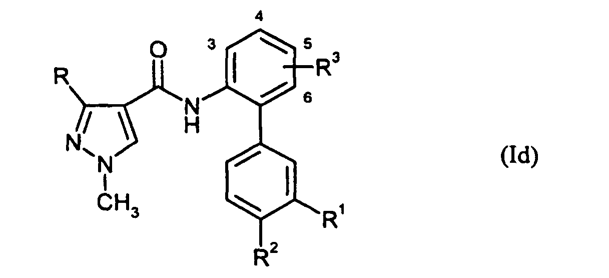 in welcher
R für Difluormethyl oder Trifluormethyl steht,
R¹ und R² unabhängig voneinander für Fluor, Chlor, Brom, Cyano, Nitro, Methyl, Ethyl, n- oder i-Propyl, n-, i-, s- oder t-Butyl, Trifluormethyl, Trichlormethyl, Trifluorethyl, Cyclopropyl, Methoxy, Ethoxy, Difluor- methoxy, Trifluormethoxy, Difluorchlormethoxy, Trifluorethoacy, Me- thylthio, Ethylthio, n- oder i-Propylthio, Difluormethylthio, Difluor- chlormethylthio, Trifluormethylthio stehen und
R³ für Fluor steht.

11. Verbindungen der Formel (Ie), 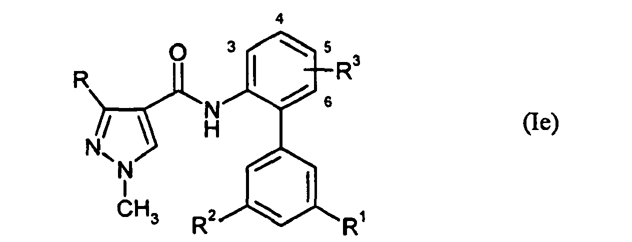 in welcher
R für Difluormethyl oder Trifluormethyl steht,
R¹ und R² unabhängig voneinander für Fluor, Chlor, Brom, Cyano, Nitro, Methyl, Ethyl, n- oder i-Propyl, n-, i-, s- oder t-Butyl, Trifluormethyl, Trichlormethyl, Trifluorethyl, Cyclopropyl, Methoxy, Ethoxy, Difluor- methoxy, Trifluormethoxy, Difluorchlormethoxy, Trifluorethoxy, Me- thylthio, Ethylthio, n- oder i-Propylthio, Difluormethylthio, Difluor- chlormethylthio, Trifluormethylthio stehen und
R³ für Fluor steht.

12. Verbindung der Formel (If)

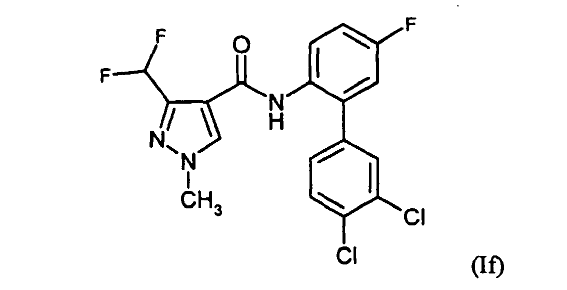

13. Verfahren zum Herstellen von Pyrazolylcarboxaniliden der Formel (I) gemäß Anspruch 1, **dadurch gekennzeichnet, dass** man
a) Pyrazolylcarbonsäurehalogenide der Formel (II) 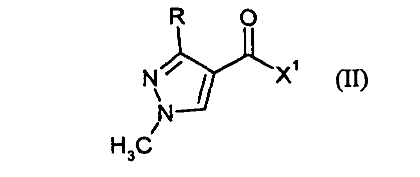 in welcher
R die in Anspruch 1 angegebenen Bedeutungen hat,
X¹ für Halogen steht,
mit Anilinderivaten der Formel (III) 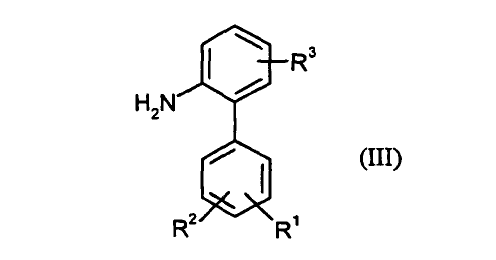 in welcher
R¹, R² und R³ die in Anspruch 1 angegebenen Bedeutungen haben,
gegebenenfalls in Gegenwart eines Säurebindemittels und gegebenenfalls in Gegenwart eines Verdünnungsmittels umsetzt, oder
b) Halogenpyrazolcarboxanilide der Formel (IV) 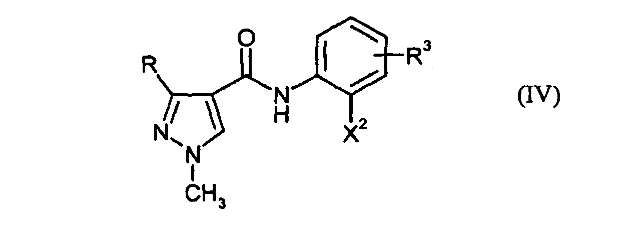 in welcher
R und R³ die in Anspruch 1 angegebenen Bedeutungen haben,
X² für Brom oder Iod steht,
mit Boronsäurederivaten der Formel (V) 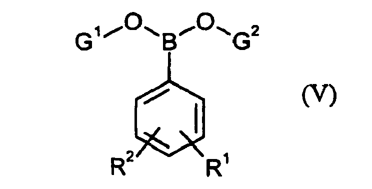 in welcher
R¹ und R² die in Anspruch 1 angegebenen Bedeutungen haben,
G¹ und G² jeweils für Wasserstoff oder zusammen für Tetramethyl- ethylen stehen,
in Gegenwart eines Katalysators, gegebenenfalls in Gegenwart eines Säurebindemittels und gegebenenfalls in Gegenwart eines Verdünnungsmittels umsetzt, oder
c) Halogenpyrazolcarboxanilide der Formel (IV) 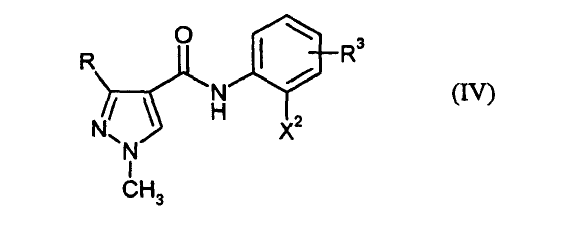 in welcher
R und R³ die in Anspruch 1 angegebenen Bedeutungen haben,
X² für Brom oder Iod steht,
in einer ersten Stufe mit einem Diboran-Derivat der Formel (VI) 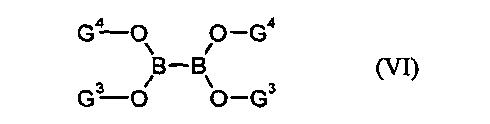 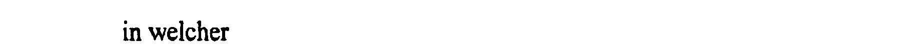
G³ und G⁴ jeweils für Alkyl oder gemeinsam für Alkandiyl stehen,
in Gegenwart eines Katalysators, gegebenenfalls in Gegenwart eines Säurebindemittels und gegebenenfalls in Gegenwart eines Verdünnungsmittels umsetzt und ohne Aufarbeitung in einer zweiten Stufe mit Halogenbenzolderivaten der Formel (VII) 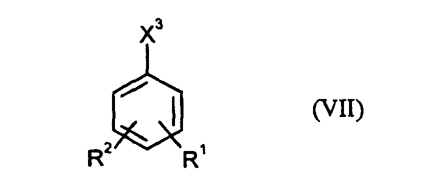 in welcher
R¹ und R² die in Anspruch 1 angegebenen Bedeutungen haben und
X³ für Brom, Iod oder Trifluormethylsulfonyloxy steht,
in Gegenwart eines Katalysators, gegebenenfalls in Gegenwart eines Säurebindemittels und gegebenenfalls in Gegenwart eines Verdünnungsmittels umsetzt.

14. Mittel zum Bekämpfen unerwünschter Mikroorganismen, **gekennzeichnet durch** einen Gehalt an mindestens einem Pyrazolylcarboxanilid der Formel (I) gemäß Anspruch 1 neben Streckmitteln und/oder oberflächenaktiven Stoffen.

15. Verwendung von Pyrazolylcarboxaniliden der Formel (I) gemäß Anspruch 1 zum Bekämpfen unerwünschter Mikroorganismen.

16. Verfahren zum Bekämpfen unerwünschter Mikroorganismen, **dadurch gekennzeichnet, dass** man Pyrazolylcarboxanilide der Formel (I) gemäß Anspruch 1 auf die Mikroorganismen und/oder deren Lebensraum ausbringt.

17. Verfahren zum Herstellen von Mitteln zum Bekämpfen unerwünschter Mikroorganismen, **dadurch gekennzeichnet, dass** man Pyrazolylcarboxanilide der Formel (I) gemäß Anspruch 1 mit Streckmitteln und/oder oberflächenaktiven Stoffen vermischt.

18. Anilinderivate der Formel (III) 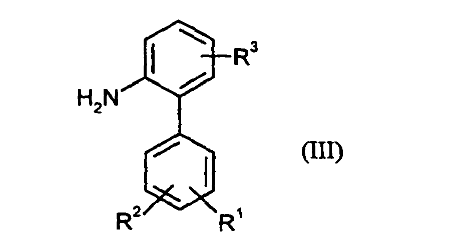 in welcher
R¹, R² und R³ die in Anspruch 1 angegebenen Bedeutungen haben.

19. Halogenpyrazolcarboxanilide der Formel (IV) 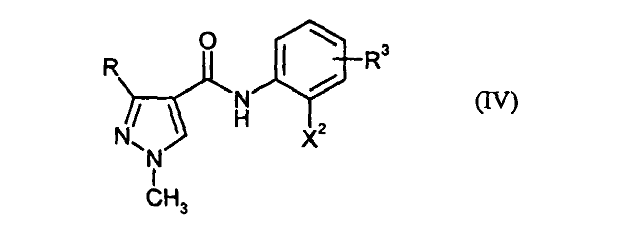 in welcher
R und R³ die in Anspruch 1 angegebenen Bedeutungen haben,
X² für Brom oder Iod steht.

20. Verwendung wenigstens einer der Verbindungen gemäß einem der Ansprüche 1 bis 12 zur Behandlung von Saatgut.

21. Saatgut, welches mit wenigstens einer der Verbindungen gemäß einem der Ansprüche 1 bis 12 behandelt wurde.

## Claims

1. Pyrazolylcarboxanilides of the formula (I) 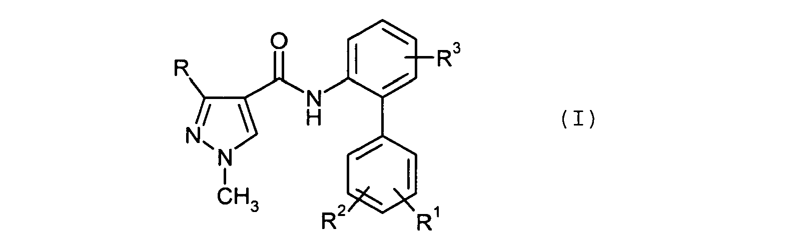 in which
R represents difluoromethyl or trifluoromethyl,
R¹ and R² independently of one another represent halogen, cyano, nitro, C₁-C₆-alkyl, C₂-C₆- alkenyl, C₁-C₄-alkoxy, C₁-C₄-alkylthio, C₁-C₄- alkylsulphonyl, C₃-C₆-cycloalkyl, or represent C₁-C₄-haloalkyl, C₁-C₄-haloalkoxy, C₁-C₄- haloalkylthio or C₁-C₄-haloalkylsulphonyl having in each case 1 to 5 halogen atoms,
R³ represents fluorine.

2. Pyrazolylcarboxanilides of the formula (I) according to Claim 1, in which
R represents difluoromethyl or trifluoromethyl,
R¹ and R² independently of one another represent fluorine, chlorine, bromine, cyano, nitro, methyl, ethyl, n- or i-propyl, n-, i-, s- or t-butyl, methoxy, ethoxy, methylthio, ethyl- thio, n- or i-propylthio, cyclopropyl, trifluoromethyl, trichloromethyl, trifluoroethyl, difluoromethoxy, trifluoromethoxy, difluorochloromethoxy, trifluoroethoxy, difluoromethylthio, difluorochloromethylthio or trifluoromethyl- thio,
R³ represents fluorine.

3. Pyrazolylcarboxanilides of the formula (I) according to Claim 1, in which
R represents difluoromethyl or trifluoromethyl,
R¹ and R² independently of one another represent fluorine, chlorine, bromine, methyl, trifluoromethyl, difluoromethoxy or trifluoromethoxy,
R³ represents fluorine.

4. Pyrazolylcarboxanilides of the formula (I) according to Claim 1 in which R¹ represents fluorine and R² represents chlorine.

5. Pyrazolylcarboxanilides of the formula (I) according to Claim 1 in which R¹ represents fluorine and R² represents fluorine.

6. Pyrazolylcarboxanilides of the formula (I) according to Claim 1 in which R¹ represents methyl or trifluoromethyl.

7. Compounds of the formula (Ia), 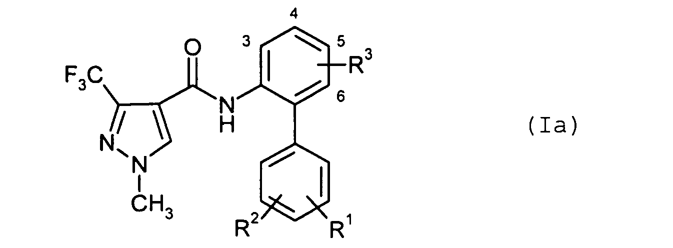 in which
R¹ and R² independently of one another represent fluorine, chlorine, bromine, cyano, nitro, methyl, ethyl, n- or i-propyl, n-, i-, s- or t-butyl, trifluoromethyl, trichloromethyl, trifluoroethyl, cyclopropyl, methoxy, ethoxy, difluoromethoxy, trifluoromethoxy, difluorochloromethoxy, trifluoroethoxy, methylthio, ethylthio, n- or i-propylthio, difluoromethylthio, difluorochloromethylthio, trifluoromethylthio and
R³ represents fluorine.

8. Compounds of the formula (Ib), 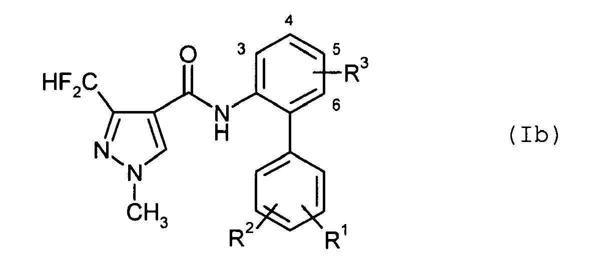 in which
R¹ and R² independently of one another represent fluorine, chlorine, bromine, cyano, nitro, methyl, ethyl, n- or i-propyl, n-, i-, s- or t-butyl, trifluoromethyl, trichloromethyl, trifluoroethyl, cyclopropyl, methoxy, ethoxy, difluoromethoxy, trifluoromethoxy, difluorochloromethoxy, trifluoroethoxy, methylthio, ethylthio, n- or i-propylthio, difluoromethylthio, difluorochloromethylthio, trifluoromethylthio and
R³ represents fluorine.

9. Compounds of the formula (Ic), 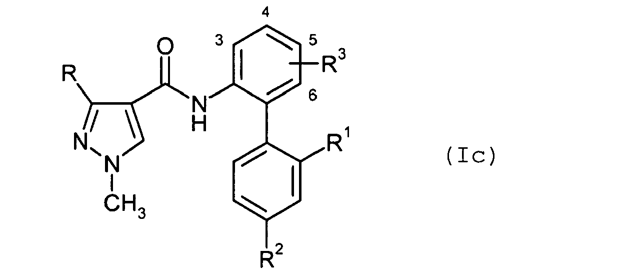 in which
R represents difluoromethyl or trifluoromethyl,
R¹ and R² independently of one another represent fluorine, chlorine, bromine, cyano, nitro, methyl, ethyl, n- or i-propyl, n-, i-, s- or t-butyl, trifluoromethyl, trichloromethyl, trifluoroethyl, cyclopropyl, methoxy, ethoxy, difluoromethoxy, trifluoromethoxy, difluorochloromethoxy, trifluoroethoxy, methylthio, ethylthio, n- or i-propylthio, difluoromethylthio, difluorochloromethylthio, trifluoromethylthio and
R³ represents fluorine.

10. Compounds of the formula (Id), 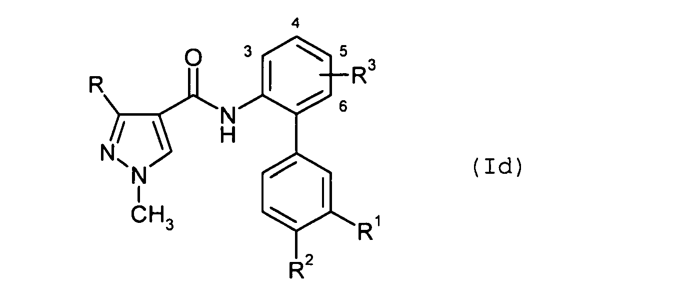 in which
R represents difluoromethyl or trifluoromethyl,
R¹ and R² independently of one another represent fluorine, chlorine, bromine, cyano, nitro, methyl, ethyl, n- or i-propyl, n-, i-, s- or t-butyl, trifluoromethyl, trichloromethyl, trifluoroethyl, cyclopropyl, methoxy, ethoxy, difluoromethoxy, trifluoromethoxy, difluorochloromethoxy, trifluoroethoxy, methylthio, ethylthio, n- or i-propylthio, difluoromethylthio, difluorochloromethylthio, trifluoromethylthio and
R³ represents fluorine.

11. Compounds of the formula (Ie), 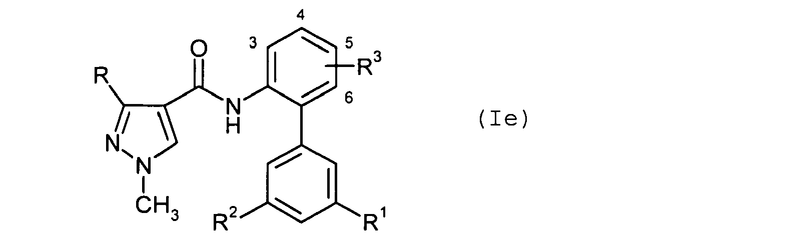 in which
R represents difluoromethyl or trifluoromethyl,
R¹ and R² independently of one another represent fluorine, chlorine, bromine, cyano, nitro, methyl, ethyl, n- or i-propyl, n-, i-, s- or t-butyl, trifluoromethyl, trichloromethyl, trifluoroethyl, cyclopropyl, methoxy, ethoxy, difluoromethoxy, trifluoromethoxy, difluorochloromethoxy, trifluoroethoxy, methylthio, ethylthio, n- or i-propylthio, difluoromethylthio, difluorochloromethylthio, trifluoromethylthio and
R³ represents fluorine.

12. Compound of the formula (If)

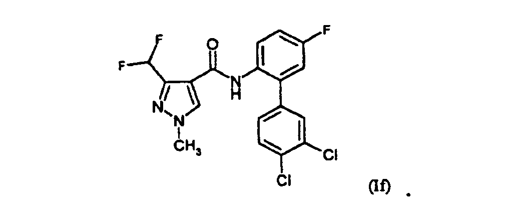

13. Process for preparing pyrazolylcarboxanilides of the formula (I) according to Claim 1, **characterized in that**
a) pyrazolylcarbonyl halides of the formula (II) 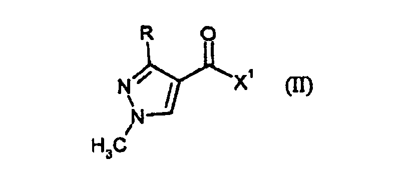 in which
R is as defined in Claim 1,
X¹ represents halogen,
are reacted with aniline derivatives of the formula (III) 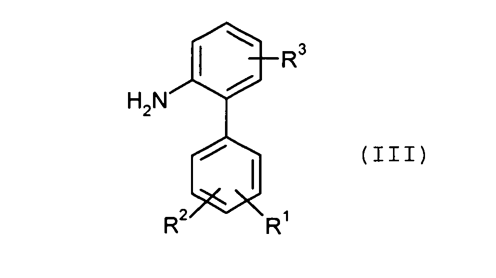 in which
R¹, R² and R³ are as defined in Claim 1,
if appropriate in the presence of an acid binder and if appropriate in the presence of a diluent, or
b) halopyrazolecarboxanilides of the formula (IV) 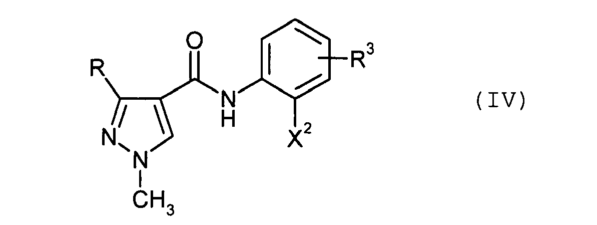 in which
R and R³ are as defined in Claim 1,
X² represents bromine or iodine,
are reacted with boronic acid derivatives of
the formula (V) 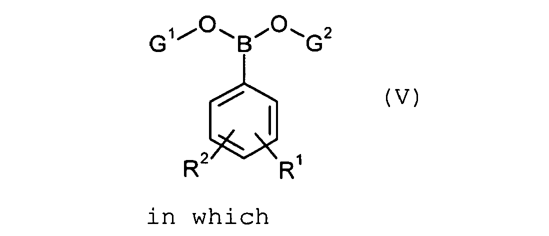
R¹ and R² are as defined in Claim 1,
G¹ and G² each represent hydrogen or together represent tetramethylethylene,
in the presence of a catalyst, if appropriate in the presence of an acid binder and if appropriate in the presence of a diluent, or
c) halopyrazolecarboxanilides of the formula (IV) 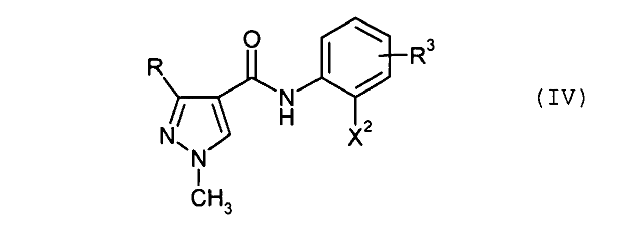 in which
R and R³ are as defined in Claim 1,
X² represents bromine or iodine,
are, in a first step, reacted with a diborane derivative of the formula (VI) 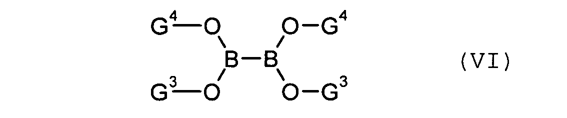 in which
G³ and G⁴ each represent alkyl or together represent alkanediyl,
in the presence of a catalyst, if appropriate in the presence of an acid binder and if appropriate in the presence of a diluent and are, without work-up, reacted in a second step with halobenzene derivatives of the formula (VII) 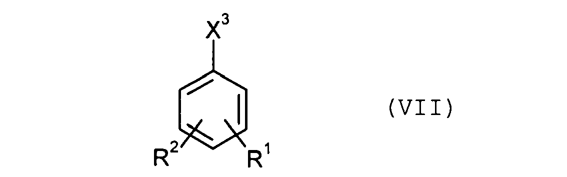 in which
R¹ and R² are as defined in Claim 1 and
X³ represents bromine, iodine or trifluoromethylsulphonyloxy,
in the presence of a catalyst, if appropriate in the presence of an acid binder and if appropriate in the presence of a diluent.

14. Compositions for controlling unwanted microorganisms, **characterized in that** they comprise at least one pyrazolylcarboxanilide of the formula (I) according to Claim 1, in addition to extenders and/or surfactants.

15. Use of pyrazolylcarboxanilides of the formula (I) according to Claim 1 for controlling unwanted microorganisms.

16. Method for controlling unwanted microorganisms, **characterized in that** pyrazolylcarboxanilides of the formula (I) according to Claim 1 are applied to the microorganisms and/or their habitat.

17. Process for preparing compositions for controlling unwanted microorganisms, **characterized in that** pyrazolylcarboxanilides of the formula (I) according to Claim 1 are mixed with extenders and/or surfactants.

18. Aniline derivatives of the formula (III) 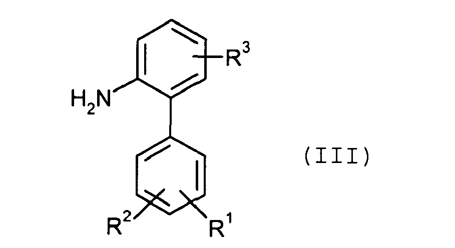 in which
R¹, R² and R³ are as defined in Claim 1.

19. Halopyrazolecarboxanilides of the formula (IV) 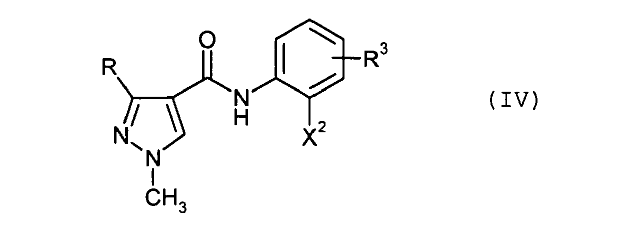 in which
R and R³ are as defined in Claim 1,
X² represents bromine or iodine.

20. Use of at least one of the compounds according to any of Claims 1 to 12 for the treatment of seed.

21. Seed which has been treated with at least one of the compounds according to any of Claims 1 to 12.

## Revendications

1. Pyrazolylcarboxanilides de formule (I) 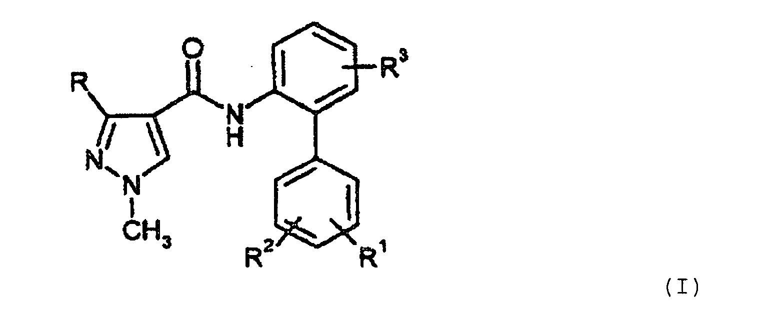 dans laquelle
R représente le groupe difluorométhyle ou trifluoro- méthyle,
R¹ et R² représentent, indépendamment l'un de l'autre, un atome d'halogène ou un groupe cyano, nitro, alkyle en C₁-C₆, alcényle en C₂-C₆, alcoxy en C₁-C₄, alkyl (C₁-C₄) thio, alkyl (C₁-C₄) sulfonyle, cycloalkyle en C₃-C₆ ou un groupe halogénoalkyle en C₁-C₄, halogénoalcoxy en C₁-C₄, halogéno- alkyl (C₁-C₄) thio ou halogénoalkyl (C₁-C₄) sulfonyle ayant chacun de 1 à 5 atomes de carbone,
R³ représente un atome de fluor.

2. Pyrazolylcarboxanilides de formule (I) selon la revendication 1, dans lesquels
R représente le groupe difluorométhyle ou trifluoro- méthyle,
R¹ et R² représentent, indépendamment l'un de l'autre, un atome de fluor, chlore, brome, un groupe cyano, nitro, méthyle, éthyle, n- ou isopropyle, n-, iso-, sec- ou tert-butyle, méthoxy, éthoxy, méthylthio, éthylthio, n- ou isopropylthio, cyclopropyle, trifluorométhyle, trichlorométhyle, trifluoroéthyle, difluorométhoxy, trifluorométhoxy, difluorochlorométhoxy, trifluoroéthoxy, difluorométhylthio, difluorochlorométhylthio ou trifluorométhylthio,
R³ représente un atome de fluor.

3. Pyrazolylcarboxanilides de formule (I) selon la revendication 1, dans lesquels
R représente le groupe difluorométhyle ou trifluoro- méthyle,
R¹ et R² représentent, indépendamment l'un de l'autre, un atome de fluor, chlore, brome, un groupe méthyle, trifluorométhyle, difluorométhoxy ou trifluorométhoxy,
R³ représente un atome de fluor.

4. Pyrazolylcarboxanilides de formule (I) selon la revendication 1, dans lesquels R¹ représente un atome de fluor et R² représente un atome de chlore.

5. Pyrazolylcarboxanilides de formule (I) selon la revendication 1, dans lesquels R¹ représente un atome de fluor et R² représente un atome de chlore.

6. Pyrazolylcarboxanilides de formule (I) selon la revendication 1, dans lesquels R¹ représente le groupe méthyle ou trifluorométhyle.

7. Composés de formule (Ia) 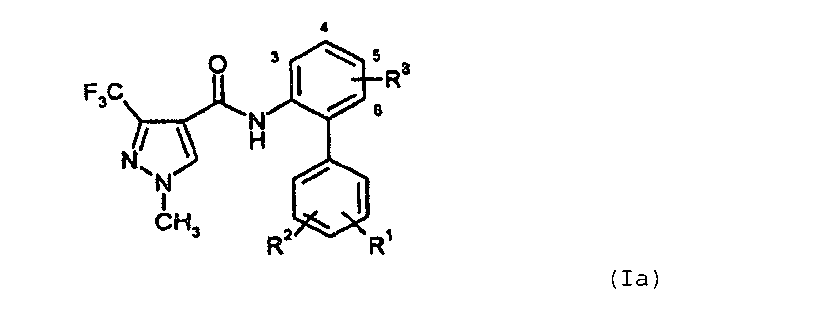 dans lesquels
R¹ et R² représentent, indépendamment l'un de l'autre, un atome de fluor, chlore, brome, un groupe cyano, nitro, méthyle, éthyle, n- ou isopropyle, n-, iso, sec- ou tert-butyle, trifluorométhyle, trichlorométhyle, trifluoroéthyle, cyclopropyle, méthoxy, éthoxy, difluorométhoxy, trifluoro- méthoxy, difluorochlorométhoxy, trifluoroéthoxy, méthylthio, éthylthio, n- ou isopropylthio, difluorométhylthio, difluorochlorométhylthio, trifluorométhylthio et
R³ représente un atome de fluor.

8. Composés de formule (Ib) 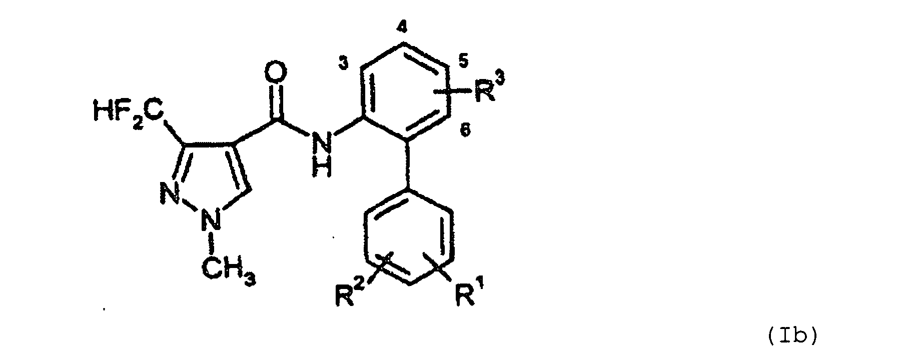 dans lesquels
R¹ et R² représentent, indépendamment l'un de l'autre, un atome de fluor, chlore, brome, un groupe cyano, nitro, méthyle, éthyle, n- ou isopropyle, n-, iso, sec- ou tert-butyle, trifluorométhyle, trichlorométhyle, trifluoroéthyle, cyclopropyle, méthoxy, éthoxy, difluorométhoxy, trifluoro- méthoxy, difluorochlorométhoxy, trifluoroéthoxy, méthylthio, éthylthio, n- ou isopropylthio, difluorométhylthio, difluorochlorométhylthio, trifluorométhylthio et
R³ représente un atome de fluor.

9. Composés de formule (Ic) 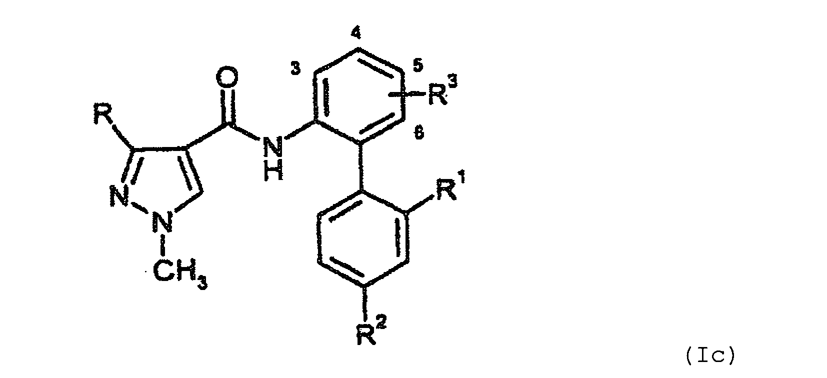 dans lesquels
R représente le groupe difluorométhyle ou trifluoro- méthyle,
R¹ et R² représentent, indépendamment l'un de l'autre, un atome de fluor, chlore, brome, un groupe cyano, nitro, méthyle, éthyle, n- ou isopropyle, n-, iso, sec- ou tert-butyle, trifluorométhyle, trichlorométhyle, trifluoroéthyle, cyclopropyle, méthoxy, éthoxy, difluorométhoxy, trifluoro- méthoxy, difluorochlorométhoxy, trifluoroéthoxy, méthylthio, éthylthio, n- ou isopropylthio, difluorométhylthio, difluorochlorométhylthio, trifluorométhylthio et
R³ représente un atome de fluor.

10. Composés de formule (Id) 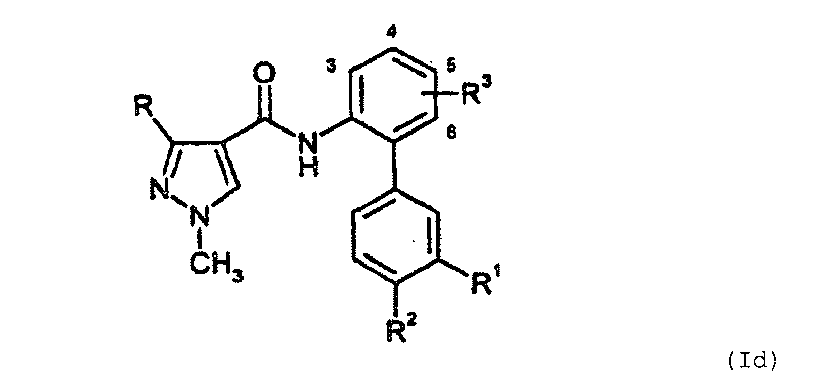 dans lesquels
R représente le groupe difluorométhyle ou trifluoro- méthyle,
R¹ et R² représentent, indépendamment l'un de l'autre, un atome de fluor, chlore, brome, un groupe cyano, nitro, méthyle, éthyle, n- ou isopropyle, n-, iso, sec- ou tert-butyle, trifluorométhyle, trichlorométhyle, trifluoroéthyle, cyclopropyle, méthoxy, éthoxy, difluorométhoxy, trifluoro- méthoxy, difluorochlorométhoxy, trifluoroéthoxy, méthylthio, éthylthio, n- ou isopropylthio, difluorométhylthio, difluorochlorométhylthio, trifluorométhylthio et
R³ représente un atome de fluor.

11. Composés de formule (Ie) 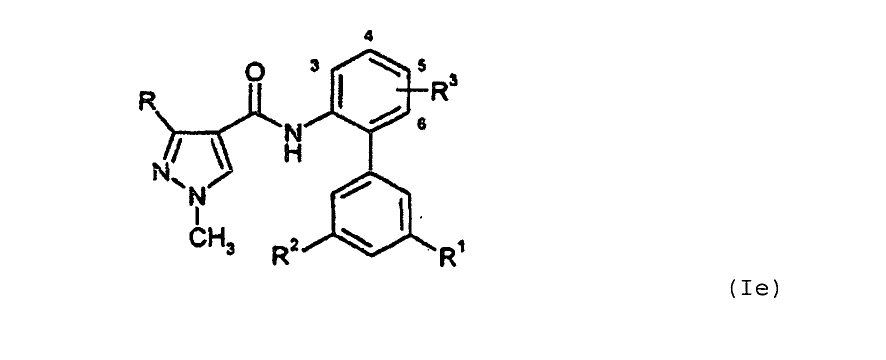 dans lesquels
R représente le groupe difluorométhyle ou trifluoro- méthyle,
R¹ et R² représentent, indépendamment l'un de l'autre, un atome de fluor, chlore, brome, un groupe cyano, nitro, méthyle, éthyle, n- ou isopropyle, n-, iso, sec- ou tert-butyle, trifluorométhyle, trichlorométhyle, trifluoroéthyle, cyclopropyle, méthoxy, éthoxy, difluorométhoxy, trifluoro- méthoxy, difluorochlorométhoxy, trifluoroéthoxy, méthylthio, éthylthio, n- ou isopropylthio, difluorométhylthio, difluorochlorométhylthio, trifluorométhylthio et
R³ représente un atome de fluor.

12. Composé de formule (If)

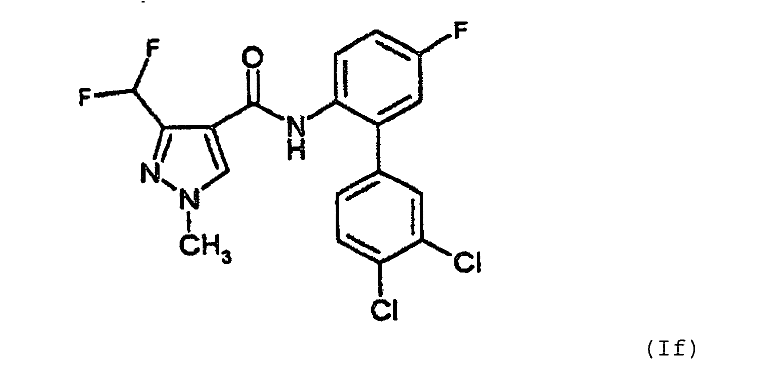

13. Procédé pour la préparation de pyrazolecarboxanilides de formule (I) selon la revendication 1, **caractérisé en ce qu'**on fait réagir
a) des halogénures de pyrazolylcarbonyle de formule (II) 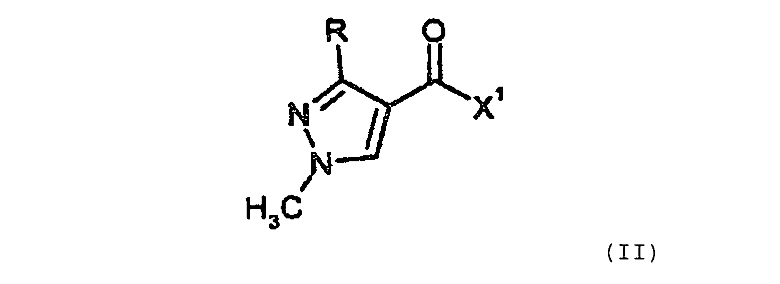 dans laquelle
R a les significations indiquées dans la revendication 1,
X¹ représente un atome d'halogène,
avec des dérivés d'anilide de formule (III) 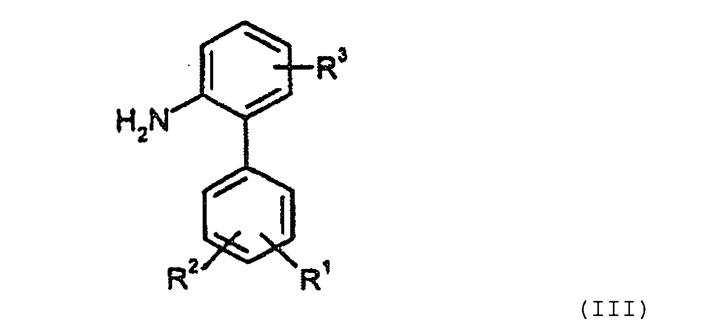 dans laquelle
R¹, R² et R³ ont les significations indiquées dans la revendication 1.
éventuellement en présence d'un agent capteur d'acide et éventuellement en présence d'un diluant, ou
b) des halogénopyrazolecarboxanilides de formule (IV) 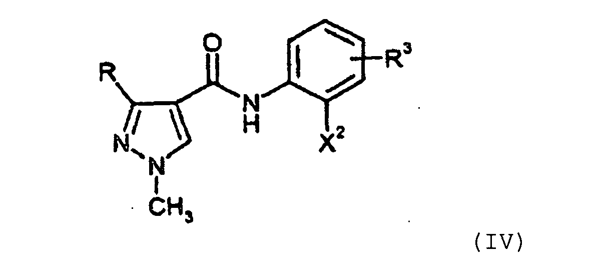 dans laquelle
R et R³ ont les significations indiquées dans la revendication 1,
X² représente un atome de brome ou d'iode,
avec des dérivés d'acide boronique de formule (V) 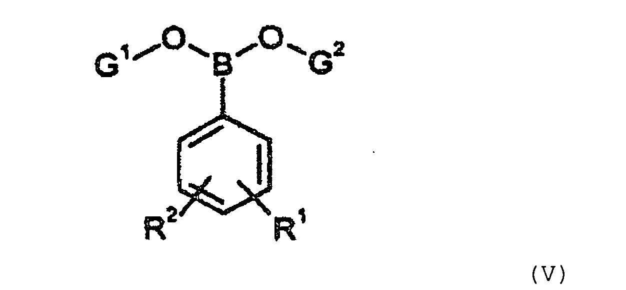 dans laquelle
R¹ et R² ont les significations indiquées dans la revendication 1,
G¹ et G² représentent chacun un atome d'hydrogène ou représentent ensemble le groupe tétraméthyléthylène,
en présence d'un catalyseur, éventuellement en présence d'un agent capteur d'acide et éventuellement en présence d'un diluant, ou
c) des halogénopyrazolecarboxanilides de formule (IV) 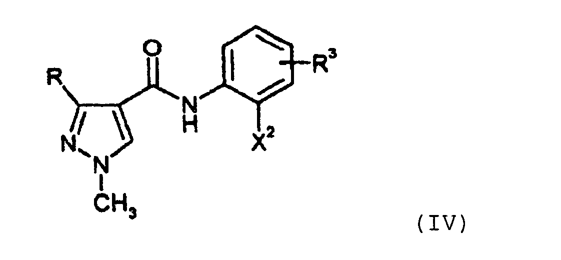 dans laquelle
R et R³ ont les significations indiquées dans la revendication 1,
X² représente un atome de brome ou d'iode,
dans une première étape avec un dérivé de diborane de formule (VI) 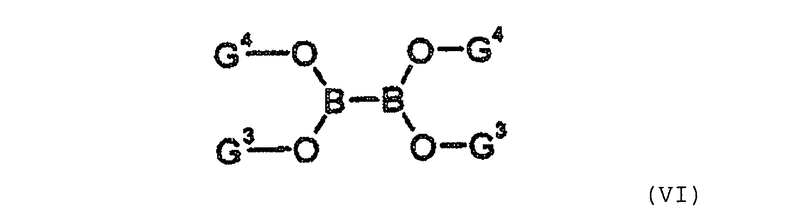 dans laquelle
G³ et G⁴ représentent chacun un groupe alkyle ou ensemble un groupe alcanediyle,
en présence d'un catalyseur, éventuellement en présence d'un agent capteur d'acide et éventuellement d'un diluant, et, sans traitement final, dans une deuxième étape, avec des des dérivés d'halogénobenzène de formule (VII) 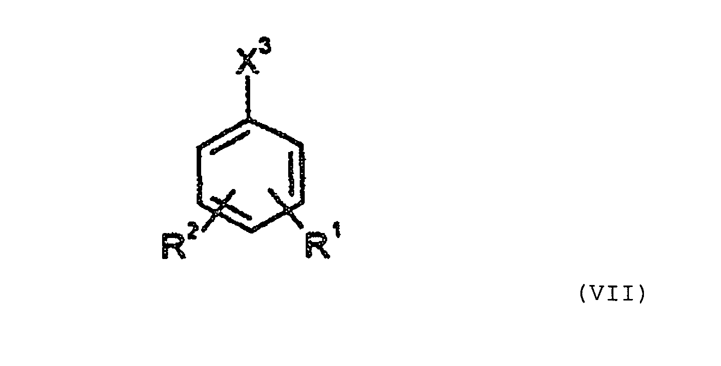 dans laquelle
R¹ et R² ont les significations indiquées dans la revendication 1 et
X³ représente un atome de brome, d'iode ou le groupe trifluorométhylsulfonyle,
en présence d'un catalyseur, éventuellement en présence d'un agent capteur d'acide et éventuellement en présence d'un diluant.

14. Composition destinée à la lutte contre des micro-organismes indésirables, **caractérisée par** une teneur en au moins un pyrazolylcarboxanilide de formule (I) selon la revendication 1, en plus d'excipients et/ou de substances tensioactives.

15. Utilisation de pyrazolecarboxanilides de formule (I) selon la revendication 1, pour la lutte contre des micro-organismes indésirables.

16. Procédé pour la lutte contre des micro-organismes indésirables, **caractérisé en ce qu'**on applique des pyrazolylcarboxanilides de formule (I) selon la revendication 1 sur les micro-organismes ou et/ou leur habitat.

17. Procédé pour la préparation de compositions destinées à la lutte contre des micro-organismes indésirables, **caractérisé en ce qu'**on mélange des pyrazolylcarboxanilides de formule (I) selon la revendication 1 avec des excipients et/ou des substances tensioactives.

18. Dérivés d'anilide de formule (III) 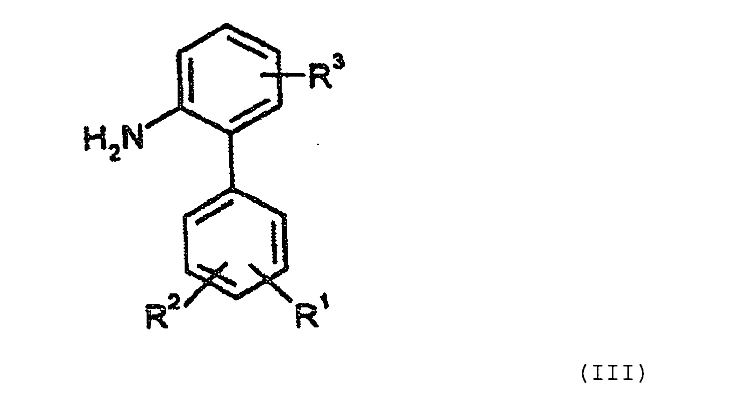 dans laquelle
R¹, R² et R³ ont les significations indiquées dans la revendication 1.

19. Halogénopyrazolecarboxanilides de formule (IV) 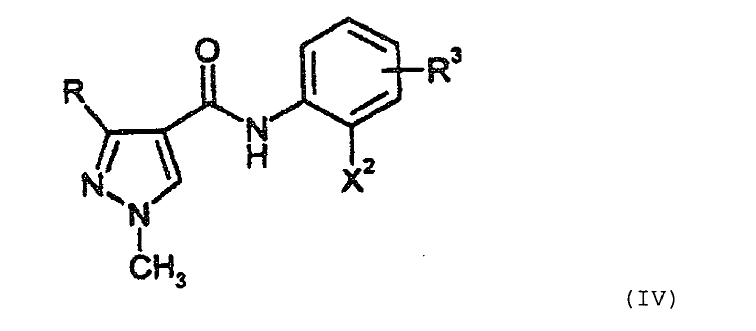 dans laquelle
R et R³ ont les significations indiquées dans la revendication 1,
X² représente un atome de brome ou d'iode.

20. Utilisation d'au moins un des composés selon l'une quelconque des revendications 1 à 12, pour le traitement de semence.

21. Semence, qui a été traitée par au moins un des composés selon l'une quelconque des revendications 1 à 12.
